# EUROPEAN PATENT SPECIFICATION

(11) **EP 3 811 940 B1**
(45) Date of publication and mention of the grant of the patent: **10.05.2023**
(21) Application number: 20202973.2
(22) Date of filing: 21.10.2020
(51) Int. Cl.: A61K 31/427, C07D 417/04, C07D 417/14, A61P 35/00

(54) **SUBSTITUTED 4,5'-BITHIAZOLES AS INHIBITORS OF THE HUMAN DNA TOPOISOMERASE II**
SUBSTITUIERTE 4,5'-BITHIAZOLE ALS INHIBITOREN DER MENSCHLICHEN DNA-TOPOISOMERASE II
4,5'-BITHIAZOLES SUBSTITUÉS COMME INHIBITEURS DE LA TOPOISOMÉRASE II DE L'ADN HUMAIN

(30) Priority: 22.10.2019 LU 101453
(43) Date of publication of application: 28.04.2021
(73) Proprietor: Kemijski Institut, 1000 Ljubljana (SI)
(72) Inventor: Perdih, Andrej, 1353 Borovnica (SI); Bergant, Kaja, 1236 Trzin (SI); Brvar, Matjaz, 1410 Zagorje ob Savi (SI); Janezic, Matej, 5000 Nova Gorica (SI)
(74) Representative: Zacco GmbH

(56) References cited:
- WO-A1-2006/125805
- WO-A2-2005/068444
- WO-A2-2009/018219
- US-A1- 2017 340 636
- BERGANT LOBODA KAJA ET AL: "Substituted 4,5'-Bithiazoles as Catalytic Inhibitors of Human DNA Topoisomerase II[alpha]", JOURNAL OF CHEMICAL INFORMATION AND MODELING, vol. 60, no. 7, 2 June 2020 (2020-06-02), pages 3662-3678, XP55777961, US
- PIALA A. T.: "Discovery of novel TAOK2 inhibitor scaffolds from high-throughput screening.", BIOORGANIC AND MEDICINAL CHEMISTRY LETTERS, vol. 26, 6 July 2016 (2016-07-06), pages 3923-3927, XP002796680,
- MORI MATTIA ET AL: "A Combination Strategy to Inhibit Pim-1: Synergism between Noncompetitive and ATP-Competitive Inhibitors", CHEMMEDCHEM, vol. 8, no. 3, 22 February 2013 (2013-02-22), pages 484-496, XP55777902, DE

## Description

### FIELD OF THE INVENTION

The present invention relates to substituted 4,5'-bithiazoles compounds as topoisomerase II catalytic inhibitors. In particular, the present invention relates to 4,5'-bithiazoles compounds of general formula (I) for use in combination with another anticancer agent in the treatment of a cancer. The present invention also relates to pharmaceutical compositions and combinations comprising 4,5'-bithiazoles compounds of general formula (I) and another anticancer agent. The invention is defined in the appended claims.

### BACKGROUND OF THE INVENTION

DNA topoisomerase II is an enzyme that catalyzes topological changes of the DNA molecule [1]. It has been found in all cell types and it is essential for cell viability. Its role includes the maintenance of intracellular DNA supercoiling, removing supercoils that build up ahead of and behind transcription and replication complexes, and the decatenation of daughter chromosomes following DNA replication. The topoisomerase II action involves cleavage of both DNA strands, transient formation of protein-DNA covalent bonds stabilizing the DNA break, passage of another segment of double stranded DNA through the enzyme-stabilized break, and re-sealing the break at the end of the catalytic process [2].

Topoisomerase II, which exists in two isoforms, alpha and beta, is an important target in cancer therapy since its disruption is lethal to proliferating tumor cells. Human topoisomerase II exists in α (170 kDa) and β (180 kDa) isoforms [3] that are products of two distinctive genes [4]. Human topo IIα represents a main target of the type II family for cancer therapies [5-8].

Topoisomerase II inhibitors are traditionally divided into two large groups. The topo II poisons act by stabilizing the covalent topo II - DNA complex, which leads to permanent breaks in the cell DNA, resulting in chromosome translocations and apoptosis [9]. They are established molecules in clinical practice with anticancer drugs such as etoposide, doxorubicin, mitoxantrone, as its prime members [9-11].

Nonetheless, the utilization of this group of drugs is linked to severe side effects, especially cardiotoxicity and induction of secondary malignancies that are directly associated with the mechanism of action of topo II poisons - the prolonged stabilization of the transient covalent topo II - DNA complex [12, 13]. Another reason for developing new anticancer drugs is the phenomenon called drug resistance. Many types of cancer can over time develop resistance to anticancer pharmaceuticals among them also topo II poisons (e.g. DNA mutations, metabolic changes) [14-17].

This stimulated the development of a new diverse group of catalytic inhibitors of human topo IIα that tries to take advantage of the available alternative inhibition paradigms. These compounds can be used as single therapeutic agents or in a combination therapy with topo II poisons for more efficient and safer treatment of cancer [12, 18, 19].

Currently, four subgroups of catalytic inhibitors are described in the literature: compounds that prevent binding of the enzyme and the DNA molecule, compounds that prevent the DNA cleavage, compounds that inhibit the ATP hydrolysis, and compounds that bind to the ATP binding site [9, 20]. More precisely, in the literature already several chemical classes are described as catalytic inhibitors of topo IIα that target the ATP binding site [20-23].

We have now found that the substituted 4,5'-bithiazoles can also be used as template for the design of compounds which act as catalytic inhibitors of human DNA topoisomerases II α/β.

Piala A. T. et al. [52] disclose thiazole compounds as TAOK2 inhibitors, while Mori et al. [53] disclose thiazole compounds as Pim-1 inhibitors. WO 2009/018219, WO 2006/125805, WO 2005/068444 and US 2017/340636 also disclose certain thiazole compounds. However, none of these prior art documents teaches or suggests substituted 4,5'-bithiazoles compounds as catalytic inhibitors of human topoisomerase II, let alone suggests a combination therapy for cancer treatment targeting same.

### SUMMARY OF THE INVENTION

The present invention provides to a 4,5'-bithiazole compound of general formula (I), or a pharmaceutically acceptable salt, solvate, amide, ester, ether, or N-oxide thereof, 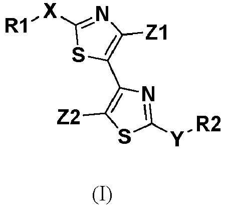
for use in combination with another anticancer agent in the treatment of a cancer;
wherein
R1 is hydrogen, -C(O)R1', substituted or unsubstituted C₁₋₆ alkyl, substituted or unsubstituted C₂₋₆ alkenyl, substituted or unsubstituted C₂₋₆ alkynyl, substituted or unsubstituted cycloalkyl, substituted or unsubstituted cycloalkenyl, substituted or unsubstituted aryl, or substituted or unsubstituted heteroaryl;
R1' is -OH, substituted or unsubstituted C₁₋₆ alkyl, substituted or unsubstituted C₂₋₆ alkenyl, substituted or unsubstituted C₂₋₆ alkynyl, substituted or unsubstituted cycloalkyl, substituted or unsubstituted cycloalkenyl, substituted or unsubstituted aryl, or substituted or unsubstituted heteroaryl;
R2 is substituted or unsubstituted aryl, or substituted or unsubstituted heteroaryl;
X is -O-, -S-, -CH₂-, or -NH-;
Y is -O-, -S-, -CH₂-, or -NH-;
Z1 is hydrogen, substituted or unsubstituted C₁₋₆ alkyl, substituted or unsubstituted C₂₋₆ alkenyl, substituted or unsubstituted C₂₋₆ alkynyl, substituted or unsubstituted cycloalkyl, substituted or unsubstituted cycloalkenyl, substituted or unsubstituted aryl, or substituted or unsubstituted heteroaryl; and
Z2 is hydrogen, substituted or unsubstituted C₁₋₆ alkyl, substituted or unsubstituted C₂₋₆ alkenyl, substituted or unsubstituted C₂₋₆ alkynyl, substituted or unsubstituted cycloalkyl, substituted or unsubstituted cycloalkenyl, substituted or unsubstituted aryl, or substituted or unsubstituted heteroaryl;
wherein the anticancer agent is selected from the group consisting of type II topoisomerase inhibitors including topoisomerase II poisons and catalytic inhibitors of topoisomerase II, type I topoisomerase inhibitors, Hsp90 inhibitors, proteasome inhibitors, inhibitors of histone deacetylases, protein kinase inhibitors, telomerase inhibitors and microtubule-interfering agents.

The present invention also provides a pharmaceutical composition comprising a compound of general formula (I) as defined herein, another anticancer agent, and a pharmaceutically acceptable carrier, adjuvant or vehicle;
wherein the anticancer agent is selected from the group consisting of type II topoisomerase inhibitors including topoisomerase II poisons and catalytic inhibitors of topoisomerase II, type I topoisomerase inhibitors, Hsp90 inhibitors, proteasome inhibitors, inhibitors of histone deacetylases, protein kinase inhibitors, telomerase inhibitors and microtubule-interfering agents.

The present invention also provides to a combination comprising i) a compound as defined herein and ii) another anticancer agent;
wherein the anticancer agent is selected from the group consisting of type II topoisomerase inhibitors including topoisomerase II poisons and catalytic inhibitors of topoisomerase II, type I topoisomerase inhibitors, Hsp90 inhibitors, proteasome inhibitors, inhibitors of histone deacetylases, protein kinase inhibitors, telomerase inhibitors and microtubule-interfering agents.

### BRIEF DESCRIPTION OF FIGURES AND TABLES

**Figure 1****:** Results of the DNA decatenation assay catalyzed by human topo IIα (1A) and topo IIβ (1B) and results of cleavage assay (1C).
**Figure 2****.** Results of various ATPase assays (2A and 2B) and result of MST bonding experiment (2C).
**Figure 3****.** Results of the MTS assay for substituted-4,5'-bithiazoles on HepG2 and MCF-7 cells.
**Figure 4****.** Cell cycle analysis, cell proliferation and induction of the DNA double stand breaks (DBS) by the substituted 4,5'-bithiazole compounds.

**Table 1:** Structures and IC₅₀ values of the substituted 4,5'-bithiazoles.
**Table 2:** Results of the decatenation assay catalysed by human topo IIα compounds **1** and **9** and positive control (topo II poison etoposide). Results are represented as % of decatenated kDNA.
**Table 3:** Results of the decatenation assay catalysed by human topo IIβ for compounds **1** and **9** and positive control (topo II poison etoposide). Results are represented as % of decatenated kDNA.
**Table 4:** % of Linear DNA, determined in cleavage assay for compounds **1, 9** and etoposide at four concentrations.
**Table 5.** IC₅₀ values for compound **1** at different concentrations of ATP with the determined R² values for the generated fits.
**Table 6:** Determined cytotoxicity of the substituted 4,5'-bithiazoles against HepG2 and MCF-7 cancer cell lines.
**Table 7:** Results of the cell cycle analysis for compound **1.** Experiments were performed in 3 independent measurements and SD values were calculated. Significant differences between the vehicle control (0) and treated cells (compound 1) as well as PC (etoposide; 50 µM) were calculated using ANOVA.

### DETAILED DESCRIPTION OF THE INVENTION

The present invention is based on substituted 4,5'-bithiazoles compounds as topoisomerase II catalytic inhibitors. It has surprisingly been found that compounds of general formula (I) inhibit the catalytic activity of human topoisomerase II, which makes them particularly useful in medicine.

The various aspects of the present invention, including the compound of general formula (I) and its various embodiments, are described in more detail below. It should become clear that any details described in connection with one aspect of the present invention applies mutatis mutandis to any other aspect of the present invention.

In one aspect, the present invention is directed to a compound of general formula (I), or a pharmaceutically acceptable salt, solvate, amide, ester, ether, or N-oxide thereof, 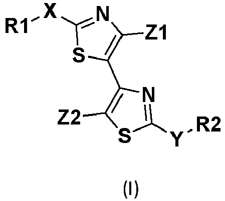 for use in combination with another anticancer agent in the treatment of a cancer; wherein
R1 is hydrogen, -C(O)R1', substituted or unsubstituted C₁₋₆ alkyl, substituted or unsubstituted C₂₋₆ alkenyl, substituted or unsubstituted C₂₋₆ alkynyl, substituted or unsubstituted cycloalkyl, substituted or unsubstituted cycloalkenyl, substituted or unsubstituted aryl, or substituted or unsubstituted heteroaryl;
R1' is -OH, substituted or unsubstituted C₁₋₆ alkyl, substituted or unsubstituted C₂₋₆ alkenyl, substituted or unsubstituted C₂₋₆ alkynyl, substituted or unsubstituted cycloalkyl, substituted or unsubstituted cycloalkenyl, substituted or unsubstituted aryl, or substituted or unsubstituted heteroaryl;
R2 is substituted or unsubstituted aryl, or substituted or unsubstituted heteroaryl;
X is -O-, -S-, -CH₂-, or -NH-;
Y is -O-, -S-, -CH₂-, or -NH-;
Z1 is hydrogen, substituted or unsubstituted C₁₋₆ alkyl, substituted or unsubstituted C₂₋₆ alkenyl, substituted or unsubstituted C₂₋₆ alkynyl, substituted or unsubstituted cycloalkyl, substituted or unsubstituted cycloalkenyl, substituted or unsubstituted aryl, or substituted or unsubstituted heteroaryl; and
Z2 is hydrogen, substituted or unsubstituted C₁₋₆ alkyl, substituted or unsubstituted C₂₋₆ alkenyl, substituted or unsubstituted C₂₋₆ alkynyl, substituted or unsubstituted cycloalkyl, substituted or unsubstituted cycloalkenyl, substituted or unsubstituted aryl, or substituted or unsubstituted heteroaryl;
wherein the anticancer agent is selected from the group consisting of type II topoisomerase inhibitors including topoisomerase II poisons and catalytic inhibitors of topoisomerase II, type I topoisomerase inhibitors, Hsp90 inhibitors, proteasome inhibitors, inhibitors of histone deacetylases, protein kinase inhibitors, telomerase inhibitors and microtubule-interfering agents.

According to certain embodiments of a compound of formula (I), R2 is a substituted or unsubstituted monocyclic aryl or substituted or unsubstituted monocyclic heteroaryl.

According to certain embodiments of a compound of formula (I), R2 is a substituted or unsubstituted monocyclic aryl, preferably is a substituted monocyclic aryl, more preferably an aryl substituted by one, two or three substituents.

According to particular embodiments of a compound of formula (I), R2 is a substituted or unsubstituted phenyl, preferably a substituted phenyl, more preferably a phenyl substituted by one, two or three substituents.

In case that the aryl or heteroaryl as defined in R2 is substituted by one or more substituents these may be independently selected from the group consisting of hydrogen, hydroxyl, substituted or unsubstituted C₁₋₆ alkyl, substituted or unsubstituted C₁₋₆ alkoxy, -C(O)R8, - S(O)₂NR9R9', and -NHCOR10_{;} wherein
R8 is -OH or substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6-membered heteroaryl, preferably is -OH or substituted or unsubstituted C₁₋₆ alkyl;
R9 is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6-membered heteroaryl, preferably is substituted or unsubstituted oxazolyl; and
R9' is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6-membered heteroaryl, preferably is hydrogen;
R10 is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6-membered heteroaryl, preferably is-CH₃.

According to certain embodiments of a compound of formula (I), the aryl or heteroaryl as defined in R2 if substituted is substituted by one or more substituents independently selected from the group consisting of -H, -OH, -CH₃, -CF₃, -COOH, -COCH₃, -SO₂NH₂, -OCH₃, -NHCOH₃, and

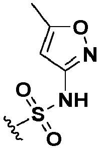

According to certain embodiments, the compound of formula (I) is a compound of formula (II) 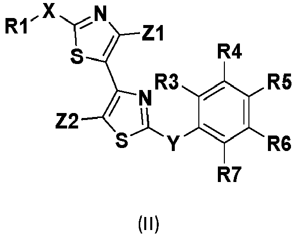 wherein
R1 is hydrogen, -C(O)R1', substituted or unsubstituted C₁₋₆ alkyl, substituted or unsubstituted C₂₋₆ alkenyl, substituted or unsubstituted C₂₋₆ alkynyl, substituted or unsubstituted cycloalkyl, substituted or unsubstituted cycloalkenyl, substituted or unsubstituted substituted or unsubstituted aryl, or substituted or unsubstituted heteroaryl;
R1' is -OH, substituted or unsubstituted C₁₋₆ alkyl, C₂₋₆ alkenyl, substituted or unsubstituted C₂₋₆ alkynyl, substituted or unsubstituted cycloalkyl, substituted or unsubstituted cycloalkenyl, substituted or unsubstituted substituted or unsubstituted aryl, or substituted or unsubstituted heteroaryl;
R3, R4, R5, R6 and R7 are independently selected from the group consisting of hydrogen, hydroxyl, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic heteroaryl, substituted or unsubstituted C₁₋₆ alkoxy, -C(O)R8, - S(O)2NR9R9', and -NHCOR10;
R8 is -OH or substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6-membered heteroaryl, preferably is -OH or substituted or unsubstituted C₁₋₆ alkyl;
R9 is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6-membered heteroaryl, preferably is substituted or unsubstituted oxazolyl;
R9' is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6-membered heteroaryl, preferably is hydrogen;
R10 is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6-membered heteroaryl, preferably is-CH₃
X is -O-, -S-, -CH₂-, or -NH-;
Y is -O-, -S-, -CH₂-, or -NH-;
Z1 is hydrogen, substituted or unsubstituted C₁₋₆ alkyl, substituted or unsubstituted C₂₋₆ alkenyl, substituted or unsubstituted C₂₋₆ alkynyl, substituted or unsubstituted cycloalkyl, substituted or unsubstituted cycloalkenyl, substituted or unsubstituted aryl, or substituted or unsubstituted heteroaryl; and
Z2 is hydrogen, substituted or unsubstituted C₁₋₆ alkyl, substituted or unsubstituted C₂₋₆ alkenyl, substituted or unsubstituted C₂₋₆ alkynyl, substituted or unsubstituted cycloalkyl, substituted or unsubstituted cycloalkenyl, substituted or unsubstituted aryl, or substituted or unsubstituted heteroaryl.

According to certain embodiments of a compound of formula (I) or (II), X is -NH-.

According to certain embodiments of a compound of formula (I) or (II), X is -O-.

According to certain embodiments of a compound of formula (I) or (II), X is -S-.

According to certain embodiments of a compound of formula (I) or (II), X is -CH₂-.

According to certain embodiments of a compound of formula (I) or (II), Y is -NH-.

According to certain embodiments of a compound of formula (I) or (II), Y is-O-.

According to certain embodiments of a compound of formula (I) or (II), Y is-S-.

According to certain embodiments of a compound of formula (I) or (II), Y is- CH₂-.

According to certain embodiments of a compound of formula (I) or (II), both X and Y are -NH-

According to certain embodiments of a compound of formula (I) or (II), Z1 is hydrogen or substituted or unsubstituted C₁₋₆ alkyl.

According to preferred embodiments of a compound of formula (I) or (II), Z1 is substituted or unsubstituted C₁₋₃ alkyl.

According to more preferred embodiments of a compound of formula (I) or (II), Z1 is substituted or unsubstituted methyl.

According to even more preferred embodiments of a compound of formula (I) or (II), Z1 is unsubstituted methyl.

According to preferred embodiments of a compound of formula (I) or (II), Z1 is hydrogen.

According to certain embodiments of a compound of formula (I) or (II), Z2 is hydrogen or substituted or unsubstituted C₁₋₆ alkyl.

According to preferred embodiments of a compound of formula (I) or (II), Z2 is substituted or unsubstituted C₁₋₃ alkyl.

According to more preferred embodiments of a compound of formula (I) or (II), Z2 is substituted or unsubstituted methyl.

According to even more preferred embodiments of a compound of formula (I) or (II), Z2 is unsubstituted methyl.

According to preferred embodiments of a compound of formula (I) or (II), Z2 is hydrogen.

According to particular embodiments, the compound of formula (I) or (II) is a compound of formula (III) 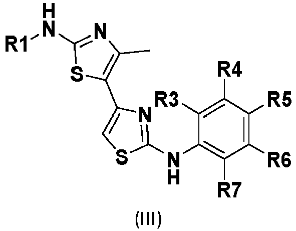 wherein
R1 is hydrogen, -C(O)R1', substituted or unsubstituted C₁₋₆ alkyl, C₂₋₆ alkenyl, substituted or unsubstituted C₂₋₆ alkynyl, substituted or unsubstituted cycloalkyl, substituted or unsubstituted cycloalkenyl, substituted or unsubstituted aryl, or substituted or unsubstituted heteroaryl;
R1' is -OH, substituted or unsubstituted C₁₋₆ alkyl, substituted or unsubstituted C₂₋₆ alkenyl, substituted or unsubstituted C₂₋₆ alkynyl, substituted or unsubstituted cycloalkyl, substituted or unsubstituted cycloalkenyl, substituted or unsubstituted aryl, or substituted or unsubstituted heteroaryl;
R3, R4, R5, R6 and R7 are independently selected from the group consisting of hydrogen, hydroxyl, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic heteroaryl, substituted or unsubstituted C₁₋₆ alkoxy, -C(O)R8, -S(O)₂NR9R9', and -NHCOR10;
R8 is -OH or substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6-membered heteroaryl, preferably is -OH or substituted or unsubstituted C₁₋₆ alkyl;
R9 is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6-membered heteroaryl, preferably is substituted or unsubstituted oxazolyl;
R9' is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6-membered heteroaryl, preferably is hydrogen; and
R10 is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6-membered heteroaryl, preferably is -CH₃.

According to certain embodiments of a compound of any one of formulae (I) to (III), R1 is hydrogen, -C(O)R1', or substituted or unsubstituted C₁₋₆ alkyl; wherein R1' is -OH, substituted or unsubstituted C₁₋₆ alkyl, substituted or unsubstituted substituted or unsubstituted aryl, or substituted or unsubstituted heteroaryl.

According to certain embodiments of a compound of any one of formulae (I) to (III), R1 is substituted or unsubstituted cycloalkyl.

According to certain embodiments of a compound of any one of formulae (I) to (III), R1 is substituted or unsubstituted cycloalkenyl.

According to certain embodiments of a compound of any one of formulae (I) to (III), R1 is substituted or unsubstituted substituted or unsubstituted aryl.

According to certain embodiments of a compound of any one of formulae (I) to (III), R1 is or substituted or unsubstituted heteroaryl.

According to certain embodiments of a compound of any one of formulae (I) to (III), R1 is hydrogen, -C(O)R1', or substituted or unsubstituted C₁₋₆ alkyl; wherein R1' is -OH or substituted or unsubstituted C₁₋₆ alkyl.

According to particular embodiments of a compound of any one of formulae (I) to (III), R1 is hydrogen.

According to particular embodiments of a compound of any one of formulae (I) to (III), R1 is - C(O)R1'.

According to particular embodiments of a compound of any one of formulae (I) to (III), R1 is substituted or unsubstituted C₁₋₆ alkyl.

According to particular embodiments of a compound of any one of formulae (I) to (III), R1 is substituted or unsubstituted C₁₋₆ alkyl, such as substituted or unsubstituted C₁₋₄ alkyl.

According to particular embodiments of a compound of any one of formulae (I) to (III), R1 is - C(O)R1', wherein R1' is -OH.

According to particular embodiments of a compound of any one of formulae (I) to (III), R1 is - C(O)R1', wherein R1' is substituted or unsubstituted C₁₋₆ alkyl, such as a substituted or unsubstituted C₁₋₄ alkyl.

According to particular embodiments of a compound of any one of formulae (I) to (III), R1 is - C(O)R1', wherein R1' is a substituted or unsubstituted C₁₋₃ alkyl, such as a substituted or unsubstituted C₁₋₂ alkyl.

According to particular embodiments of a compound of any one of formulae (I) to (III), R1 is - COCH₃.

According to particular embodiments of a compound of any one of formulae (I) to (III), R1 is - COCH₂CH₃.

According to certain embodiments of a compound of any one of formulae (II) or (III), R3, R4, R5, R6 and R7 are independently selected from the group consisting of hydrogen, hydroxyl, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic heteroaryl, substituted or unsubstituted C₁₋₆ alkoxy, - C(O)R8, -S(O)₂NR9R9', and -NHCOR10; wherein
R8 is -OH or substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6- membered heteroaryl, preferably is-OH or substituted or unsubstituted C₁₋₆ alkyl;
R9 is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6- membered heteroaryl, such as substituted or unsubstituted oxazolyl,
R9' is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6- membered heteroaryl, preferably is hydrogen;
R10 is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6- membered heteroaryl, preferably is - CH₃.

According to certain embodiments of a compound of any one of formulae (II) or (III), R3, R4, R5, R6 and R7 are independently selected from the group consisting of hydrogen, hydroxyl, substituted or unsubstituted C₁₋₄ alkyl, substituted or unsubstituted C₁₋₄ alkoxy, -C(O)R8, - S(O)₂NR9R9', and -NHCOR10; wherein R8 is -OH or substituted or unsubstituted C₁₋₄ alkyl, R9 is hydrogen, substituted or unsubstituted C₁₋₄ alkyl, or substituted or unsubstituted monocyclic 5-membered heteroaryl, such as substituted or unsubstituted oxazolyl, R9' is hydrogen, and R10 is -CH₃.

According to certain embodiments of a compound of any one of formulae (II) or (III), R3, R4, R5, R6 and R7 are independently selected from the group consisting of -H, -OH, -CH₃, -CF₃, -COOH, - COCH₃, -SO₂NH₂, -OCH₃, -NHCOCH₃, and

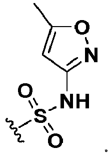

According to certain embodiments of a compound of any one of formulae (II) or (III), R5 is selected from the group consisting of hydrogen, hydroxyl, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic heteroaryl, substituted or unsubstituted C₁₋₆ alkoxy, -C(O)R8, -S(O)₂NR9R9', and -NHCOR10; wherein
R8 is -OH or substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6- membered heteroaryl, preferably is-OH or substituted or unsubstituted C₁₋₆ alkyl;
R9 is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6- membered heteroaryl, such as substituted or unsubstituted oxazolyl,
R9' is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6- membered heteroaryl, preferably is hydrogen; and
R10 is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6- membered heteroaryl, preferably is - CH₃.

According to certain embodiments of a compound of any one of formulae (II) or (III), R5 is selected from the group consisting of hydrogen, hydroxyl, substituted or unsubstituted C₁₋₄ alkyl, substituted or unsubstituted C₁₋₄ alkoxy, -C(O)R8, -S(O)₂NR9R9', and -NHCOCH₃; wherein R8 is - OH or substituted or unsubstituted C₁₋₄ alkyl, R9 is hydrogen, substituted or unsubstituted C₁₋₄ alkyl, or substituted or unsubstituted monocyclic 5-membered heteroaryl, such as substituted or unsubstituted oxazolyl, and R9' is hydrogen.

According to certain embodiments of a compound of any one of formulae (II) or (III), R5 is hydrogen.

According to certain embodiments of a compound of any one of formulae (II) or (III), R5 is hydroxyl.

According to certain embodiments of a compound of any one of formulae (II) or (III), R5 is substituted or unsubstituted C₁₋₆ alkyl, such as substituted or unsubstituted C₁₋₄ alkyl.

According to certain embodiments of a compound of any one of formulae (II) or (III), R5 is substituted or unsubstituted C₂₋₆ alkenyl.

According to certain embodiments of a compound of any one of formulae (II) or (III), R5 is substituted or unsubstituted C₂₋₆ alkynyl.

According to certain embodiments of a compound of any one of formulae (II) or (III), R5 is substituted or unsubstituted monocyclic cycloalkyl.

According to certain embodiments of a compound of any one of formulae (II) or (III), R5 is substituted or unsubstituted monocyclic aryl.

According to certain embodiments of a compound of any one of formulae (II) or (III), R5 is substituted or unsubstituted monocyclic heteroaryl,

According to certain embodiments of a compound of any one of formulae (II) or (III), R5 is substituted or unsubstituted C₁₋₄ alkoxy.

According to certain embodiments of a compound of any one of formulae (II) or (III), R5 is - C(O)R8.

According to certain embodiments of a compound of any one of formulae (II) or (III), R5 is - S(O)₂NR9R9'.

According to certain embodiments of a compound of any one of formulae (II) or (III), R5 is - NHCOR10.

According to certain embodiments of a compound of any one of formulae (II) or (III), R5 is selected from the group consisting of -H, -OH, -CH₃, -CF₃, -COOH, -COCH₃, -SO₂NH₂, -OCH₃, - NHCOCH₃, and

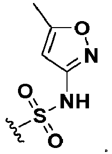

According to certain embodiments of a compound of any one of formulae (II) or (III), R3 is selected from the group consisting of hydrogen, hydroxyl, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic heteroaryl, substituted or unsubstituted C₁₋₆ alkoxy, -C(O)R8, -S(O)₂NR9R9', and -NHCOR10; wherein
R8 is -OH or substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6- membered heteroaryl, preferably is-OH or substituted or unsubstituted C₁₋₆ alkyl;
R9 is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6- membered heteroaryl, such as substituted or unsubstituted oxazolyl,
R9' is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6- membered heteroaryl, preferably is hydrogen, and
R10 is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6- membered heteroaryl, preferably is - CH₃.

According to certain embodiments of a compound of any one of formulae (II) or (III), R3 is selected from the group consisting of hydrogen, hydroxyl, substituted or unsubstituted C₁₋₄ alkyl, substituted or unsubstituted C₁₋₄ alkoxy, -C(O)R8, -S(O)₂NR9R9', and -NHCOCH₃; wherein R8 is - OH or substituted or unsubstituted C₁₋₄ alkyl, R9 is hydrogen, substituted or unsubstituted C₁₋₄ alkyl, or substituted or unsubstituted monocyclic 5-membered heteroaryl, such as substituted or unsubstituted oxazolyl, and R9' is hydrogen.

According to certain embodiments of a compound of any one of formulae (II) or (III), R3 is selected from the group consisting of -H, -OH, -CH₃, -CF₃, -COOH, -COCH₃, -SO₂NH₂, -OCH₃, - NHCOCH₃, and

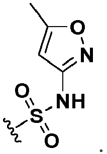

According to certain embodiments of a compound of any one of formulae (II) or (III), R3 is -H, - OH or -COOH.

According to certain embodiments of a compound of any one of formulae (II) or (III), R3 is -H.

According to certain embodiments of a compound of any one of formulae (II) or (III), R3 is -OH.

According to certain embodiments of a compound of any one of formulae (II) or (III), R3 is -COOH.

According to certain embodiments of a compound of any one of formulae (II) or (III), R4 is selected from the group consisting of hydrogen, hydroxyl, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic heteroaryl, substituted or unsubstituted C₁₋₆ alkoxy, -C(O)R8, -S(O)₂NR9R9', and -NHCOR10; wherein
R8 is -OH or substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6- membered heteroaryl, preferably is-OH or substituted or unsubstituted C₁₋₆ alkyl;
R9 is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6- membered heteroaryl, such as substituted or unsubstituted oxazolyl,
R9' is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6- membered heteroaryl, preferably is hydrogen, and
R10 is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6- membered heteroaryl, preferably is - CH₃.

According to certain embodiments of a compound of any one of formulae (II) or (III), R4 is selected from the group consisting of hydrogen, hydroxyl, substituted or unsubstituted C₁₋₄ alkyl, substituted or unsubstituted C₁₋₄ alkoxy, -C(O)R8, -S(O)₂NR9R9', and -NHCOCH₃; wherein R8 is - OH or substituted or unsubstituted C₁₋₄ alkyl, R9 is hydrogen, substituted or unsubstituted C₁₋₄ alkyl, or substituted or unsubstituted monocyclic 5-membered heteroaryl, such as substituted or unsubstituted oxazolyl, and R9' is hydrogen.

According to certain embodiments of a compound of any one of formulae (II) or (III), R4 is selected from the group consisting of -H, -OH, -CH₃, -CF₃, -COOH, -COCH₃, -SO₂NH₂, -OCH₃, - NHCOCH₃, and

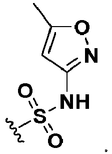

According to certain embodiments of a compound of any one of formulae (II) or (III), R4 is -H, - OH or -COOH.

According to certain embodiments of a compound of any one of formulae (II) or (III), R4 is -H.

According to certain embodiments of a compound of any one of formulae (II) or (III), R4 is -OH.

According to certain embodiments of a compound of any one of formulae (II) or (III), R4 is -COOH.

According to certain embodiments of a compound of any one of formulae (II) or (III), R6 is selected from the group consisting of hydrogen, hydroxyl, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic heteroaryl, substituted or unsubstituted C₁₋₆ alkoxy, -C(O)R8, -S(O)₂NR9R9', and -NHCOR10; wherein
R8 is -OH or substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6- membered heteroaryl, preferably is-OH or substituted or unsubstituted C₁₋₆ alkyl;
R9 is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6- membered heteroaryl, such as substituted or unsubstituted oxazolyl,
R9' is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6- membered heteroaryl, preferably is hydrogen, and
R10 is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6- membered heteroaryl, preferably is - CH₃.

According to certain embodiments of a compound of any one of formulae (II) or (III), R6 is selected from the group consisting of hydrogen, hydroxyl, substituted or unsubstituted C₁₋₆ alkyl, substituted or unsubstituted C₁₋₆ alkoxy, -C(O)R8, -S(O)₂NR9R9', and -NHCOR10; wherein
R8 is -OH or substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6- membered heteroaryl, preferably is-OH or substituted or unsubstituted C₁₋₆ alkyl;
R9 is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6- membered heteroaryl, such as substituted or unsubstituted oxazolyl,
R9' is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6- membered heteroaryl, preferably is hydrogen; and
R10 is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6- membered heteroaryl, preferably is - CH₃.

According to certain embodiments of a compound of any one of formulae (II) or (III), R6 is selected from the group consisting of hydrogen, hydroxyl, substituted or unsubstituted C₁₋₄ alkyl, substituted or unsubstituted C₁₋₄ alkoxy, -C(O)R8, -S(O)₂NR9R9', and -NHCOCH₃; wherein R8 is - OH or substituted or unsubstituted C₁₋₄ alkyl, R9 is hydrogen, substituted or unsubstituted C₁₋₄ alkyl, or substituted or unsubstituted monocyclic 5-membered heteroaryl, such as substituted or unsubstituted oxazolyl, and R9' is hydrogen.

According to certain embodiments of a compound of any one of formulae (II) or (III), R6 is selected from the group consisting of -H, -OH, -CH₃, -CF₃, -COOH, -COCH₃, -SO₂NH₂, -OCH₃, - NHCOCH₃, and

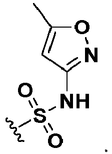

According to certain embodiments of a compound of any one of formulae (II) or (III), R6 is -H, - OH or -COOH.

According to certain embodiments of a compound of any one of formulae (II) or (III), R6 is -H.

According to certain embodiments of a compound of any one of formulae (II) or (III), R6 is -OH.

According to certain embodiments of a compound of any one of formulae (II) or (III), R6 is -COOH.

According to certain embodiments of a compound of any one of formulae (II) or (III), R7 is selected from the group consisting of hydrogen, hydroxyl, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic heteroaryl, substituted or unsubstituted C₁₋₆ alkoxy, -C(O)R8, -S(O)₂NR9R9', and -NHCOR10; wherein
R8 is -OH or substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6- membered heteroaryl, preferably is-OH or substituted or unsubstituted C₁₋₆ alkyl;
R9 is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6- membered heteroaryl, such as substituted or unsubstituted oxazolyl,
R9' is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6- membered heteroaryl, preferably is hydrogen, and
R10 is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6- membered heteroaryl, preferably is - CH₃.

According to certain embodiments of a compound of any one of formulae (II) or (III), R7 is selected from the group consisting of hydrogen, hydroxyl, substituted or unsubstituted C₁₋₆ alkyl, substituted or unsubstituted C₁₋₆ alkoxy, -C(O)R8, -S(O)₂NR9R9', and -NHCOR10; wherein
R8 is -OH or substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6- membered heteroaryl, preferably is-OH or substituted or unsubstituted C₁₋₆ alkyl;
R9 is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6- membered heteroaryl, such as substituted or unsubstituted oxazolyl,
R9' is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6- membered heteroaryl, preferably is hydrogen; and
R10 is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6- membered heteroaryl, preferably is - CH₃.

According to certain embodiments of a compound of any one of formulae (II) or (III), R7 is selected from the group consisting of hydrogen, hydroxyl, substituted or unsubstituted C₁₋₄ alkyl, substituted or unsubstituted C₁₋₄ alkoxy, -C(O)R8, -S(O)₂NR9R9', and -NHCOCH₃; wherein R8 is - OH or substituted or unsubstituted C₁₋₄ alkyl, R9 is hydrogen, substituted or unsubstituted C₁₋₄ alkyl, or substituted or unsubstituted monocyclic 5-membered heteroaryl, such as substituted or unsubstituted oxazolyl, and R9' is hydrogen.

According to certain embodiments of a compound of any one of formulae (II) or (III), R7 is selected from the group consisting of -H, -OH, -CH₃, -CF₃, -COOH, -COCH₃, -SO₂NH₂, -OCH₃, - NHCOH₃, and

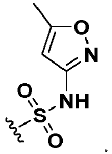

According to certain embodiments of a compound of any one of formulae (II) or (III), R7 is -H or-CH₃.

According to certain embodiments of a compound of any one of formulae (II) or (III), R7 is -H.

According to certain embodiments of a compound of any one of formulae (II) or (III), R7 is -CH₃.

According to certain embodiments of a compound of any one of formulae (II) or (III), both R6 and R7 are -H.

According to certain embodiments of a compound of any one of formulae (II) or (III), both R3 and R4 are -H.

According to certain embodiments of a compound of any one of formulae (II) or (III), each of R3, R4, R6 and R7 is -H.

According to certain embodiments of a compound of any one of formulae (II) or (III), R8 is -OH.

According to certain embodiments of a compound of any one of formulae (II) or (III), R8 is substituted or unsubstituted C₁₋₆ alkyl, such as substituted or unsubstituted C₁₋₄ alkyl.

According to certain embodiments of a compound of any one of formulae (II) or (III), R8 is substituted or unsubstituted C₁₋₃ alkyl, such as substituted or unsubstituted C₁₋₂ alkyl.

According to certain embodiments of a compound of any one of formulae (II) or (III), R9 is hydrogen.

According to certain embodiments of a compound of any one of formulae (II) or (III), R9 is substituted or unsubstituted C₁₋₆ alkyl, such as substituted or unsubstituted C₁₋₄ alkyl.

According to certain embodiments of a compound of any one of formulae (II) or (III), R9 is substituted or unsubstituted C₁₋₃ alkyl, such as substituted or unsubstituted C₁₋₂ alkyl.

According to certain embodiments of a compound of any one of formulae (II) or (III), R9 is substituted or unsubstituted monocyclic aryl, such as substituted or unsubstituted phenyl.

According to certain embodiments of a compound of any one of formulae (II) or (III), R9 is substituted or unsubstituted monocyclic 5- or 6-membered heteroaryl, such as substituted or unsubstituted oxazolyl.

According to certain embodiments of a compound of any one of formulae (II) or (III), R9' is hydrogen.

According to particular embodiments, a compound of any one of formulae (I) to (III) is a compound selected from the group consisting of: 4'-methyl-N2-(4-(trifluoromethyl)phenyl)-[4,5'-bithiazole]-2,2'-diamine, 4-((2'-amino-4'-methyl-[4,5'-bithiazol]-2-yl)amino)-3-methylphenol, 1-(4-((2'-amino-4'-methyl-[4,5'-bithiazol]-2-yl)amino)phenyl)ethan-1-one, 4-((2'-amino-4'-methyl-[4,5'-bithiazol]-2-yl)amino)-N-(5-methylisoxazol-3-yl)benzenesulfonamide, 4-((2'-amino-4'-methyl-[4,5'-bithiazol]-2-yl)amino)benzenesulfonamide, 4-((2'-amino-4'-methyl-[4,5'-bithiazol]-2-yl)amino)-2-hydroxybenzoic acid, N-(2-((3-hydroxyphenyl)amino)-4'-methyl-[4,5'-bithiazol]-2'-yl)acetamide, 4-((2'-acetamido-4'-methyl-[4,5'-bithiazol]-2-yl)amino)benzoic acid, N-(4-((2'-acetamido-4'-methyl-[4,5'-bithiazol]-2-yl)amino)phenyl)acetamide, N-(2-((4-methoxyphenyl)amino)-4'-methyl-[4,5'-bithiazol]-2'-yl)acetamide, 3-((2'-acetamido-4'-methyl-[4,5'-bithiazol]-2-yl)amino)benzoic acid, N-(2-((4-hydroxyphenyl)amino)-4'-methyl-[4,5'-bithiazol]-2'-yl)acetamide, 4-((4'-methyl-2'-propionamido-[4,5'-bithiazol]-2-yl)amino)benzoic acid, N-(4'-methyl-2-(phenylamino)-[4,5'-bithiazol]-2'-yl)acetamide, and 4-((2'-benzamido-4'-methyl-[4,5'-bithiazol]-2-yl)amino)benzoic acid.

According to particular embodiments, a compound of any one of formulae (I) to (III) is a compound selected from the group consisting of: 4'-methyl-N2-(4-(trifluoromethyl)phenyl)-[4,5'-bithiazole]-2,2'-diamine, 4-((2'-amino-4'-methyl-[4,5'-bithiazol]-2-yl)amino)-3-methylphenol, 1-(4-((2'-amino-4'-methyl-[4,5'-bithiazol]-2-yl)amino)phenyl)ethan-1-one, 4-((2'-amino-4'-methyl-[4,5'-bithiazol]-2-yl)amino)-N-(5-methylisoxazol-3-yl)benzenesulfonamide, 4-((2'-amino-4'-methyl-[4,5'-bithiazol]-2-yl)amino)benzenesulfonamide, 4-((2'-amino-4'-methyl-[4,5'-bithiazol]-2-yl)amino)-2-hydroxybenzoic acid, N-(2-((3-hydroxyphenyl)amino)-4'-methyl-[4,5'-bithiazol]-2'-yl)acetamide, 4-((2'-acetamido-4'-methyl-[4,5'-bithiazol]-2-yl)amino)benzoic acid, N-(4-((2'-acetamido-4'-methyl-[4,5'-bithiazol]-2-yl)amino)phenyl)acetamide, N-(2-((4-methoxyphenyl)amino)-4'-methyl-[4,5'-bithiazol]-2'-yl)acetamide, 3-((2'-acetamido-4'-methyl-[4,5'-bithiazol]-2-yl)amino)benzoic acid, N-(2-((4-hydroxyphenyl)amino)-4'-methyl-[4,5'-bithiazol]-2'-yl)acetamide, 4-((4'-methyl-2'-propionamido-[4,5'-bithiazol]-2-yl)amino)benzoic acid and N-(4'-methyl-2-(phenylamino)-[4,5'-bithiazol]-2'-yl)acetamide.

According to more particular embodiments, a compound of any one of formulae (I) to (III) is a compound selected from the group consisting of: 4'-methyl-N2-(4-(trifluoromethyl)phenyl)-[4,5'-bithiazole]-2,2'-diamine, N-(2-((3-hydroxyphenyl)amino)-4'-methyl-[4,5'-bithiazol]-2'-yl)acetamide, N-(4-((2'-acetamido-4'-methyl-[4,5'-bithiazol]-2-yl)amino)phenyl)acetamide, N-(2-((4-methoxyphenyl)amino)-4'-methyl-[4,5'-bithiazol]-2'-yl)acetamide, N-(4'-methyl-2-(phenylamino)-[4,5'-bithiazol]-2'-yl)acetamide.

A cancer treatable according to the present invention include, but not limited to, brain cancer, breast cancer, lung cancer, prostate cancer, colorectal cancer, melanoma, bladder cancer, lymphoma such as non-Hodgkin lymphoma, kidney cancer, endometrial cancer, leukemia, pancreatic cancer, bone cancer, gastrointestinal cancer, cervical cancer, endometrial cancer, gallbladder cancer, mouth cancer, neuroblastoma, pituitary tumor, retinoblastoma, various types of sarcoma, throat cancer, urethral cancer, vaginal cancer, ovarian cancer, fallopian tube cancer, bile duct cancer and thyroid cancer.

Another aspect of the present invention pertains to a pharmaceutical composition which comprises a compound of general formula (I) as detailed herein or a pharmaceutically acceptable salt thereof, another anticancer agent and a pharmaceutically acceptable carrier, adjuvant or vehicle; wherein the anticancer agent is selected from the group consisting of type II topoisomerase inhibitors including topoisomerase II poisons and catalytic inhibitors of topoisomerase II, type I topoisomerase inhibitors, Hsp90 inhibitors, proteasome inhibitors, inhibitors of histone deacetylases, protein kinase inhibitors, telomerase inhibitors and microtubule-interfering agents.

As a general remark, the use of "comprising" and "comprises" as used herein, especially when defining the contents of a medicament or a pharmaceutical formulation is to be understood as also disclosing "consisting of" and "consists of" respectively etc. Thus, this also includes that the contents of the respective medicament or pharmaceutical formulation are then to be also understood to be limited to the exact contents preceded by this "comprising" or "comprises" etc.

Examples of pharmaceutical compositions include any solid (tablets, pills, capsules, granules etc.) or liquid (solutions, suspensions or emulsions) composition for oral, topical or parenteral administration.

In a preferred embodiment the pharmaceutical compositions are in oral form, either solid or liquid. Suitable dose forms for oral administration may be tablets, capsules, syrops or solutions and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated accordingto methods well known in normal pharmaceutical practice, in particular with an enteric coating.

The pharmaceutical compositions may also be adapted for parenteral administration, such as sterile solutions, suspensions or lyophilized products in the appropriate unit dosage form. Adequate excipients can be used, such as bulking agents, buffering agents or surfactants.

The mentioned formulations will be prepared using standard methods such as those described or referred to in the Spanish and US Pharmacopoeias and similar reference texts.

Administration of the compounds, compositions and combinations as detailed herein may be by any suitable method, such as intravenous infusion, oral preparations, and intraperitoneal and intravenous administration. Oral administration is preferred because of the convenience for the patient and the chronic character of the diseases to be treated.

Generally, an effective administered amount of a compound as detailed herein will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.1 to 1000 mg/kg/day.

Thus, in another aspect the present invention pertains to a combination comprising i) a compound of general formula (I) as detailed herein and ii) another anticancer agent selected from the group consisting of type II topoisomerase inhibitors including topoisomerase II poisons and catalytic inhibitors of topoisomerase II, type I topoisomerase inhibitors, Hsp90 inhibitors, proteasome inhibitors, inhibitors of histone deacetylases, protein kinase inhibitors, telomerase inhibitors and microtubule-interfering agents.

According to certain embodiments, a combination comprises i) a compound of general formula (I) as detailed herein and ii) a type II topoisomerase inhibitor.

According to certain embodiments, a combination comprises i) a compound of general formula (I) as detailed herein and ii) a topoisomerase II poison.

The topoisomerase II poison employed in accordance with the present invention may be an anthracycline or an epipodophyllotoxin.

The anthracycline may, for example, be a compound selected from the group consisting of doxorubicin, idarubicin, epirubicin, aclarubicin, mitoxantrone, dactinomycin, bleomycin, mitomycin, carubicin, pirarubicin, daunorubicin, daunomycin, 4-iodo-4-deoxy-doxorubicin, N,N-dibenzyl-daunomycin, morpholinodoxorubicin, aclacinomycin, duborimycin, menogaril, nogalamycin, zorubicin, marcellomycin, detorubicin, annamycin, 7-cyanoquinocarcinol, deoxydoxorubicin, valrubicin, GPX-100, MEN-10755, and KRN5500.

The epipodophyllotoxin may, for example, be a compound selected from the group consisting of etoposide, etoposide phosphate, teniposide, tafluposide, VP-16213, and NK-611.

According to certain embodiments, the topoisomerase II poison employed in accordance with the present invention is etoposide.

According to certain embodiments, a combination comprises i) a compound of general formula (I) as detailed herein and ii) a catalytic inhibitor of topoisomerase II.

Catalytic inhibitor of topoisomerase II may, for example, be a compound selected from the group consisting of ICRF-193 and genistein.

According to certain embodiments, a combination comprises i) a compound of general formula (I) as detailed herein and ii) a type I topoisomerase inhibitor, such as a compound selected from the group consisting of irinotecan, topotecan, camptothecin, belotecan, indotecan and indimitecan.

According to certain embodiments, a combination comprises i) a compound of general formula (I) as detailed herein and ii) a Hsp90 inhibitor, such as a compound selected from the group consisting of luminespib, retaspimycin, ganetespib and NVP-AUY922.

According to certain embodiments, a combination comprises i) a compound of general formula (I) as detailed herein and ii) a proteasome inhibitor, such as a compound selected from the group consisting of bortezomib, carfilzomib and ixazomib.

According to certain embodiments, a combination comprises i) a compound of general formula (I) as detailed herein and ii) an inhibitor of histone deacetylases, such as a compound selected from the group consisting of vorinostat, romidepsin, belinostat, panabiostat, entinostat and tacedinaline.

According to certain embodiments, a combination comprises i) a compound of general formula (I) as detailed herein and ii) a protein kinase inhibitor, such as a compound selected from the group consisting of idelalisib, fasudil, temsirulimus, ribociclib, idelalisib, gefitinib, erlotinib, sunitinib, midostaurin, imatinib, infigratinib, alvocidib and dovitinib.

According to certain embodiments, a combination comprises i) a compound of general formula (I) as detailed herein and ii) a telomerase inhibitor, such as a compound selected from telomestatin and BIBR-1532.

According to certain embodiments, a combination comprises i) a compound of general formula (I) as detailed herein and ii) a microtubule-interfering agent, such as a compound selected from the group consisting of vincristine, colchicine, docetaxel and paclitaxel.

### Certain definitions

In the context of this invention, alkyl is understood as meaning saturated, linear or branched hydrocarbons, which may be unsubstituted or substituted once or several times. It encompasses e.g. -CH₃ and -CH₂-CH₃. In these radicals, C₁₋₂-alkyl represents C1- or C2-alkyl, C₁₋₃-alkyl represents C1-, C2- or C3-alkyl, C₁₋₄-alkyl represents C1-, C2-, C3- or C4-alkyl, C₁₋₅-alkyl represents C1-, C2-, C3-, C4-, or C5-alkyl, C₁₋₆-alkyl represents C1-, C2-, C3-, C4-, C5- or C6-alkyl. The alkyl radicals are preferably methyl, ethyl, propyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, if substituted also CHF₂, CF₃ or CH₂OH etc. Preferably alkyl is understood in the context of this invention as C₁₋₆ alkyl like methyl, ethyl, propyl, butyl, pentyl, hexyl, or heptyl; preferably is C₁₋₄ alkyl like methyl, ethyl, propyl, or butyl; more preferably is C₁₋₂ alkyl.

Alkenyl is understood as meaning unsaturated, linear or branched hydrocarbons, which may be unsubstituted or substituted once or several times. It encompasses groups like e.g. -CH=CH-CH₃. The alkenyl radicals are preferably vinyl (ethenyl), allyl (2-propenyl). Preferably in the context of this invention alkenyl is C₂₋₆-alkenyl like ethylene, propylene, butylene, pentylene, or hexylene; or is C₂₋₄-alkenyl, like ethylene, propylene, or butylenes.

Alkynyl is understood as meaning unsaturated, linear or branched hydrocarbons, which may be unsubstituted or substituted once or several times. It encompasses groups like e.g. -C≡C-CH₃ (1-propinyl). Preferably alkynyl in the context of this invention is C₂₋₆-alkynyl like ethyne, propyne, butyene, pentyne, or hexyne; or is C₂₋₄-alkynyl like ethyne, propyne, butyene, pentyne, or hexyne.

In the context of this invention alkoxy is understood as meaning an -O-alkyl.

In connection with alkyl, alkenyl, alkynyl and alkoxy - unless defined otherwise - the term "substituted" in the context of this invention is understood as meaning replacement of at least one hydrogen radical on a carbon atom by halogen (F, Cl, Br, I), -OH, -CN, -SH or -NH₂. More than one replacement on the same molecule and also on the same carbon atom is possible with the same or different substituents. This includes for example 3 hydrogens being replaced on the same C atom, as in the case of CF₃, or at different places of the same molecule, as in the case of e.g. - CH(OH)-CH=CH-CHCl₂.

In the context of this invention cycloalkyl is understood as meaning saturated cyclic hydrocarbons (without a heteroatom in the ring), which can be unsubstituted or once or several times substituted. Furthermore, C₃₋₄-cycloalkyl represents C3- or C4-cycloalkyl, C₃₋₅-cycloalkyl represents C3-, C4- or C5-cycloalkyl, C₃₋₆-cycloalkyl represents C3-, C4-, C5- or C6-cycloalkyl, C₃₋₇-cycloalkyl represents C3-, C4-, C5-, C6- or C7-cycloalkyl, C₃₋₈-cycloalkyl represents C3-, C4-, C5-, C6-, C7- or C8-cycloalkyl, C₄₋₅-cycloalkyl represents C4- or C5-cycloalkyl, C₄₋₆-cycloalkyl represents C4-, C5- or C6-cycloalkyl, C₄₋₇-cycloalkyl represents C4-, C5-, C6- or C7-cycloalkyl, C₅₋₆-cycloalkyl represents C5- or C6-cycloalkyl and C₅₋₇-cycloalkyl represents C5-, C6- or C7-cycloalkyl. Examples are cyclopropyl, 2-methylcyclopropyl, cyclopropylmethyl, cyclobutyl, cyclopentyl, cyclopentylmethyl, cyclohexyl, cycloheptyl, and cyclooctyl. Preferably in the context of this invention cycloalkyl is C₃₋₈cycloalkyl like cyclopropyl, cyclobutyl, cyclopentyl, cyclohexyl, cycloheptyl, or cyclooctyl; or is C₃₋₇cycloalkyl like cyclopropyl, cyclobutyl, cyclopentyl, cyclohexyl, or cycloheptyl; or is C₃₋₆cycloalkyl like cyclopropyl, cyclobutyl, cyclopentyl or cyclohexyl, especially cyclopentyl or cyclohexyl.

Preferably, the cycloalkyl is a monocyclic cycloalkyl. More preferably the cycloalkyl is a 3, 4, 5, 6, 7 or 8 membered monocyclic cycloalkyl. Even more preferably the cycloalkyl is a 3, 4, 5 or 6 membered monocyclic cycloalkyl.

In the context of this invention cycloalkenyl is understood as meaning unsaturated (but not aromatic) cyclic hydrocarbons (without a heteroatom in the ring), which can be unsubstituted or once or several times substituted. Furthermore, C₃₋₄-cycloalkenyl represents C3- or C4-cycloalkenyl, C₃₋₅-cycloalkenyl represents C3-, C4- or C5-cycloalkenyl, C₃₋₆-cycloalkenyl represents C3-, C4-, C5- or C6-cycloalkenyl, C₃₋₇-cycloalkenyl represents C3-, C4-, C5-, C6- or C7-cycloalkyl, C₃₋₈-cycloalkenyl represents C3-, C4-, C5-, C6-, C7- or C8-cycloalkenyl, C₄₋₅-cycloalkenyl represents C4- or C5-cycloalkenyl, C₄₋₆-cycloalkenyl represents C4-, C5- or C6-cycloalkyl, C₄₋₇-cycloalkenyl represents C4-, C5-, C6- or C7-cycloalkenyl, C₅₋₆-cycloalkenyl represents C5- or C6-cycloalkenyl and C₅₋₇-cycloalkenyl represents C5-, C6- or C7-cycloalkenyl. Examples are cyclopropenyl, 2-methylcyclopropenyl, cyclopropenylmethyl, cyclobutenyl, cyclopentenyl, cyclopentenylmethyl, cyclohexenyl, cycloheptenyl, and cyclooctenyl. Preferably in the context of this invention cycloalkenyl is C₃₋₈cycloalkenyl like cyclopropenyl, cyclobutenyl, cyclopentenyl, cyclohexenyl, cycloheptenyl, or cyclooctenyl; or is C₃₋₇cycloalkenyl like cyclopropenyl, cyclobutenyl, cyclopentenyl, cyclohexenyl, or cycloheptenyl; or is C₃₋₆cycloalkenyl like cyclopropenyl, cyclobutenyl, cyclopentenyl or cyclohexenyl, especially cyclopentenyl or cyclohexenyl.

Preferably, the cycloalkenyl is a monocyclic cycloalkenyl. More preferably the cycloalkenyl is a 3, 4, 5, 6, 7 or 8 membered monocyclic cycloalkenyl. Even more preferably the cycloalkenyl is a 3, 4, 5 or 6 membered monocyclic cycloalkenyl.

Aryl is understood as meaning ring systems with at least one aromatic ring but without heteroatoms even in only one of the rings. Examples are phenyl, naphthyl, fluoranthenyl, fluorenyl, tetralinyl or indanyl, in particular 9H-fluorenyl or anthracenyl radicals, which can be unsubstituted or once or several times substituted. Preferably aryl is understood in the context of this invention as phenyl, naphtyl or anthracenyl. Preferably, the aryl is a monocyclic aryl, more preferably the aryl is a 5, 6 or 7 membered monocyclic aryl, even more preferably is phenyl.

Heteroaryl (being equivalent to heteroaromatic radicals or aromatic heterocyclyls) is an aromatic heterocyclic ring system of one or more rings of which at least one aromatic ring contains one or more heteroatoms from the group consisting of nitrogen, oxygen and/or sulfur in the ring; preferably is an aromatic heterocyclic ring system of one or two rings of which at least one aromatic ring contains one or more heteroatoms from the group consisting of nitrogen, oxygen and/or sulfur in the ring. Examples include furan, benzofuran, thiophene, benzothiophene, pyrrole, pyridine, pyrimidine, pyrazine, quinoline, isoquinoline, phthalazine, benzothiazole, indole, benzotriazole, carbazole, quinazoline, thiazole, imidazole, pyrazole, oxazole, thiophene and benzimidazole. Preferably, the heteroaryl is a monocyclic heteroaryl.

Preferably, the heteroaryl is a monocyclic heteroaryl. More preferably the heteroaryl is a 5, 6 or 7 membered monocyclic heteroaryl. Even more preferably the heteroaryl is a 5 or 6 membered monocyclic heteroaryl.

In connection with cycloalkyl, cycloalkenyl, aryl or heteroaryl "substituted" is understood - unless defined otherwise - as meaning substitution of the ring-system of the cycloalkyl, cycloalkenyl, aryl or heteroaryl by halogen (F, Cl, Br, I), -OH, -CN, -SH, -NH₂, substituted or unsubstituted C₁₋₆ alkyl, substituted or unsubstituted C₁₋₆ alkoxy, -C(O)R8, -S(O)₂NR9R9', or -NHCOR10. Preferably, the term "substituted" is understood as meaning substitution of the ring-system of the cycloalkyl, cycloalkenyl, aryl or heteroaryl by halogen (F, Cl, Br, I), -OH, -CN, -SH or -NH₂. More than one replacement on the same molecule and also on the same carbon or hetero atom is possible with the same or different substituents.

The term "pharmaceutically acceptable salts" refers to those salts which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid and the like, in particular hydrochloric acid, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, N-acetylcystein and the like. In addition, these salts may be prepared by addition of an inorganic base or an organic base to the free acid. Salts derived from an inorganic base include, but are not limited to, the sodium, potassium, lithium, ammonium, calcium, magnesium salts and the like. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, N-ethylpiperidine, piperidine, polyimine resins and the like. Particular pharmaceutically acceptable salts of compounds of formula (I) are the hydrochloride salts, methanesulfonic acid salts and citric acid salts.

"Pharmaceutically acceptable esters" means that compounds of general formula (I) may be derivatised at carboxylic or hydroxy groups to provide derivatives which are capable of conversion back to the parent compounds in vivo. Examples of such compounds include physiologically acceptable and metabolically labile ester derivatives, such as methoxymethyl esters, methylthiomethyl esters and pivaloyloxymethyl esters. Additionally, any physiologically acceptable equivalents of the compounds of general formula (I), similar to the metabolically labile esters, which are capable of producing the parent compounds of general formula (I) in vivo, are within the scope of this invention.

The term "solvate" means any form of the active compound according to the invention in which this compound has attached to it via non-covalent binding another molecule (most likely a polar solvent). Especially preferred examples include hydrates and alcoholates, like methanolates or ethanolates.

The term "conjugate" is used in its broadest sense and encompasses those derivative in which the compound of the invention is linked to another molecule, such as an antibody (preferably a monoclonal antibody), polymer and the like. In a preferred embodiment, the conjugate is an antibody-drug conjugate (ADC).

A "topoisomerase inhibitor" is a chemical compound that blocks the action of topoisomerase (topoisomerase I and II). Topoisomerase inhibitors are often divided according to which type of enzyme it inhibits. Type I topoisomerase inhibitors are chemical compounds that block the action of topoisomerase I, which is important in changing topological changes in DNA molecule with inducing DNA single-strand breaks. Type II topoisomerase inhibitors are chemical compounds block the action of topoisomerase II. Type II topoisomerase inhibitors are split into two main classes: topoisomerase II poisons and catalytic inhibitors of topoisomerase II.

A "topoisomerase II poison" is a compound, including any pharmaceutically acceptable salt, solvate or amide thereof, which generally inhibits the human topoisomerase II by stabilizing the covalent cleavage complex formed between the enzyme and DNA during the catalytic cycle.

A "catalytic inhibitor of topoisomerase II" is a compound, including including any pharmaceutically acceptable salt, solvate or amide thereof, which generally disrupt catalytic turnover of topoisomerase II.

Hsp90 inhibitors are chemical substances that inhibit the activity of the Hsp90 heat shock protein, which is a chaperone protein that assists other proteins to fold properly, stabilizes proteins against heat stress, and aids in protein degradation.

Proteasome inhibitors are chemical compounds that block action proteasomes, cellular complexes that break down proteins.

Inhibitors of histone deacetylases are chemical compounds that inhibit histone deacetylases, which are enzymes that remove acetyl groups from an ε-N-acetyl lysine amino acid on a histone, allowing the histones to wrap the DNA more tightly.

Protein kinase inhibitors are chemical compounds that blocks the action of one or more protein kinases. Protein kinases are enzymes that add a phosphate group to a protein, and can modulate its function.

Telomerase inhibitors are chemical compounds that inhibit telomerase, unique reverse transcriptase enzyme which is mainly responsible to regulate the telomere length.

Microtubule-interfering agents are chemical compounds that influence the dynamic of microtubules, which are polymers that play crucial roles in a large number of cellular functions.

### Examples

### 1. In vitro investigation of inhibition properties of substituted 4,5'-bithiazoles on human DNA topoisomerase IIα

The 14 selected compounds from the class of substituted 4,5'-bithiazoles were experimentally assayed if they inhibit catalytic activity of human topo IIα using an established high-throughput screening (HTS) relaxation assay for human topo IIα [24].

The HTS assays primarily served to select compounds for further evaluation. Screening of the hit compounds was performed at four different compound concentrations and to validate the assay, etoposide was used as a control compound, for which we experimentally determined the IC₅₀ value 41.6 µM, in comparison to 60.3 µM, reported in the literature [25, 26].

**Table 1: Structures and IC₅₀ values of topo IIα inhibition of the substituted 4,5'-bithiazoles.**

| 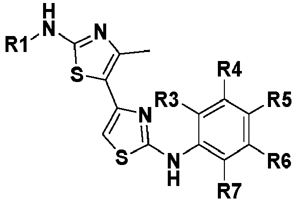 | | | | | | | |
|---|---|---|---|---|---|---|---|
| **Compound** | **R1** | **R3** | **R4** | **R5** | **R6** | **R7** | **IC₅₀ (µM)** |
| **1** | -H | -H | -H | -CF₃ | -H | -H | 33.4 |
| **2** | -H | -H | -H | -OH | -H | CH₃ | 35.8 |
| **3** | -H | -H | -H | -COCH₃ | -H | -H | 222.4 |
| **4** | -H | -H | -H | 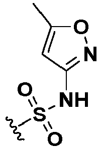 | -H | -H | 30.1 |
| **5** | -H | -H | -H | -SO₂NH₂ | -H | -H | 70.1 |
| **6** | -H | -H | -H | -COOH | -OH | -H | 37.7 |
| **7** | -COCH₃ | -H | -H | -H | -OH | -H | 33.7 |
| **8** | -COCH₃ | -H | -H | -COOH | -H | -H | 119.7 |
| **9** | -COCH₃ | -H | -H | -NHCOH₃ | -H | -H | 60.0 |
| **10** | -COCH₃ | -H | -H | -OCH₃ | -H | -H | 22.7 |
| **11** | -COCH₃ | -H | -H | -H | COOH | -H | 134.1 |
| **12** | -COCH₃ | -H | -H | -OH | -H | -H | 192.9 |
| **13** | -COCH₂CH₃ | -H | -H | -COOH | -H | -H | 123.0 |
| **14** | -COCH₃ | -H | -H | -H | -H | -H | 357.1 |
| **15** | -C(O)-phenyl | -H | -H | -COOH | -H | -H | 12,6 |

Results of the initial HTS relaxation assay are available in Table 1. Compounds showed activity on topo IIα and the determined IC₅₀ values were in the same range as the drug etoposide molecule.

In addition, these compounds possess favourable drug-like properties such as logP values below or equal to 5 and topological surface areas (TPSA) lower than 120 for all compounds, except for compound **4.**

To investigate if compounds from the class of substituted 4,5'-bithiazoles inhibit the DNA decatenation, catalysed by human topo IIα, we performed kDNA decatenation for compounds **1, 9** and etoposide as a control compound. Decatenation assay was performed in duplicates as described in Experimental Methods section using kinetoplast (kDNA) as a substrate and etoposide as a standard compound.

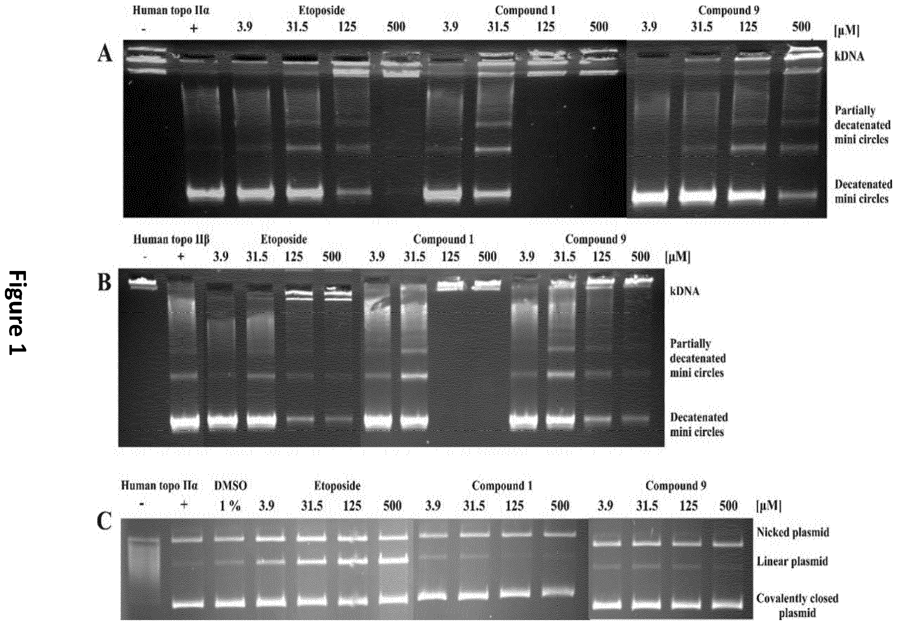

The results of decatenation assay for example compounds **1** and **9** are presented in Figure 1A. As shown in Figure 1A, tested compounds significantly inhibited the decatenation of kDNA, catalysed by human topo IIα in a concentration-dependent manner (Table 2). Etoposide reference topo II inhibitor exhibited the inhibition of human topo IIα decatenation activity with significant levels of inhibition between 500 and 125 µM, with % of inhibition 95.6 and 67.2 %, respectively. Compound **1** was more potent, with full inhibition by 125 µM and 500 µM and 19.1 % of inhibition at 31.5 µM. Compound **9** was also found to be a strong inhibitor albeit slightly less active than compound **1** with 67.3 % of inhibition at 500 µM and 11.9 % of inhibition at 125 µM. These results confirmed the significant influence of 4,5'-bithiazoles class on the catalytic activity of human topo IIα with compound **1** exhibiting higher inhibitory activity against the human topo IIα decatenation as the etoposide standard.

We also performed the decatenation assay, catalysed by human topo IIβ, to investigate the selectivity against both isoforms of human topo II for two compounds - **1** and **9.** in Figure 1B, tested compounds significantly inhibited the decatenation of kDNA, catalysed by human topo IIα in a concentration-dependent manner (Table 3). Results showed that compounds **1** fully inhibited human topo IIβ at 500 µM and 125 µM concentrations and exhibited 20.8 % inhibition at 31.5 µM. Compound **9** showed 88.6 % of inhibition at 500 µM and 73.9 % of inhibition at 125 µM (Figure 1B). This was comparable to the inhibition observed for the topo IIα isoform.

Non selectivity for both topo II isoforms could be even desired (see below) in the case of catalytic topo IIα inhibitors, that bind to the ATP binding site, if we take in consideration the similarity of ATP binding sites of both isoenzymes, observed with the alignment of crystal structure of ATPase domain of human topo IIα and homology model of ATPase domain of human topo IIβ [22]. It was previously shown that etoposide-induced carcinogenesis involves mainly the topo IIβ rather than the topo IIα isozyme [27]. Additionally, doxorubicin- induced cardiotoxicity is also connected with causing double stranded-breaks in topo IIα isoform [28]. Experiments in mice have demonstrated that topo II alpha but not beta is essential for cell proliferation [29, 30] , but recent SiRNA data shows that beta isoform can compensate alpha depletion in certain cell lines [31]. Partial compensation should be taken into consideration when developing novel catalytic inhibitors, so inhibition of both isoforms should be required [22]. Toposomerase IIβ can be useful as anticancer target also when targeting the non-proliferative cells as well as cancer stem cells. Toposomerase IIβ was recently suggested as a potent target to counteract resistance of glioblastoma cells in glioblastoma therapy [32].

**Figure 1****:** (**A**) Compounds **1** and **9** inhibited the DNA decatenation catalysed by human topo IIα. Each reaction contained the same amount of human topo IIα and the solvent DMSO (1 %), and the designated compound concentration, except the control reactions of kDNA, which had no compounds and were performed in the presence of topo IIα (+) and in the absence of topo IIα (-). Etoposide was used as a standard compound. (**B**) Compounds **1** and **9** inhibited DNA decatenation catalysed by human topo IIβ. Each reaction contained the same amount of human topo IIα and the solvent DMSO (1 %), and designated compound concentration, except the control reaction of kDNA which had no compounds and were performed in the presence of topo IIβ (+) and in the absence of topo IIα (-). Etoposide topo II poison was used as a standard compound. (**C**) Results of the cleavage assay for etoposide and compounds (**1, 9**) at four concentrations (3.9, 31.5, 125 and 500 µM). Unlike the positive control etoposide, tested compounds do not function as human topo IIα poison as they do not significantly increase the levels of linear DNA.

**Table 2: Results of decatenation assay catalysed by human topo IIα compounds 1 and 9 and positive control (etoposide). Results are represented as % of decatenated kDNA.**

| **Compound** | **% Decatenated Assay 1** | | | | **% Decatenated Assay 2** | | | | **% Decatenated Average** | | | |
|---|---|---|---|---|---|---|---|---|---|---|---|---|
| | **Concentration (µM)** | | | | **Concentration (µM)** | | | | **Concentration (µM)** | | | |
| | **3.9** | **31.5** | **125** | **500** | **3.9** | **31.5** | **125** | **500** | **3.9** | **31.5** | **125** | **500** |
| **Etoposide** | 100 | 91 | 21.6 | 1.5 | 100 | 96.7 | 44 | 7.2 | 100 | 93.9 | 32.8 | 4.4 |
| **Cp 1** | 100 | 72.4 | 0 | 0 | 91 | 89.4 | 0 | 0 | 95.5 | 80.5 | 0 | 0 |
| **Cp 9** | 98.7 | 99.4 | 89.5 | 34 | 99.2 | 97.9 | 86.7 | 31.3 | 98.9 | 98.7 | 88.1 | 32.7 |

**Table 3: Results of the decatenation assay catalysed by human topo IIβ for compounds 1 and 9 and positive control etoposide. Results are represented as % of decatenated kDNA.**

| **Compound** | **% Decatenated Assay 1** | | | | **% Decatenated Assay 2** | | | | **% Decatenated Average** | | | |
|---|---|---|---|---|---|---|---|---|---|---|---|---|
| | **Concentration (µM)** | | | | **Concentration (µM)** | | | | **Concentration (µM)** | | | |
| | **3.9** | **31.5** | **125** | **500** | **3.9** | **31.5** | **125** | **500** | **3.9** | **31.5** | **125** | **500** |
| **Etoposide** | 100.0 | 94.0 | 24.6 | 150 | 100.0 | 92.8 | 23.1 | 14.4 | 1000 | 93.4 | 23.9 | 14.7 |
| **Cp 1** | 87.3 | 78.6 | 0 | 0 | 95.5 | 79.7 | 0 | 0 | 91.3 | 79.2 | 0 | 0 |
| **Cp 9** | 87.0 | 80.1 | 30.5 | 17.5 | 75.6 | 73.3 | 21.7 | 5.3 | 81.3 | 76.7 | 26.1 | 11.4 |

To determine if compounds act as topo II poisons, cleavage assay was performed for compounds **1** and **9.** After treatment with human topo IIα, the negatively supercoiled plasmid was incubated with four different concentrations (3.9, 31.5, 125 and 500 µM) of investigated compounds (**1, 9**) and etoposide (control).

The obtained results (Figure 1C) clearly show the poison activity of etoposide, which increased amount of linear DNA with increased concentrations of the drug. In contrast, the same titration with tested compounds did not result in a significant amount of linear DNA above that of background level, which indicates that they are not topoisomerase poisons. % of Linear DNA, determined in cleavage assay for compounds **1, 9** and etoposide at four concentrations is represented in Table 4.

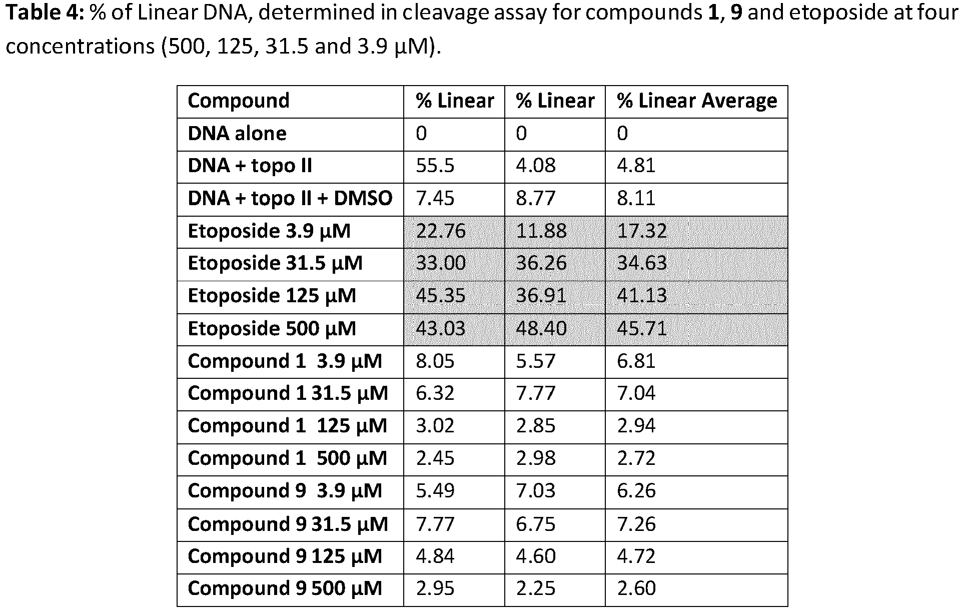

To investigate if our compounds can inhibit the ATP hydrolysis, catalysed by human topo IIα, first ATP hydrolysis assay was performed for compound **1.** This assay is coupled to the oxidation of NADH, which was indicated by the decrease of optical density at 340 nm. Etoposide was used as a control compound.

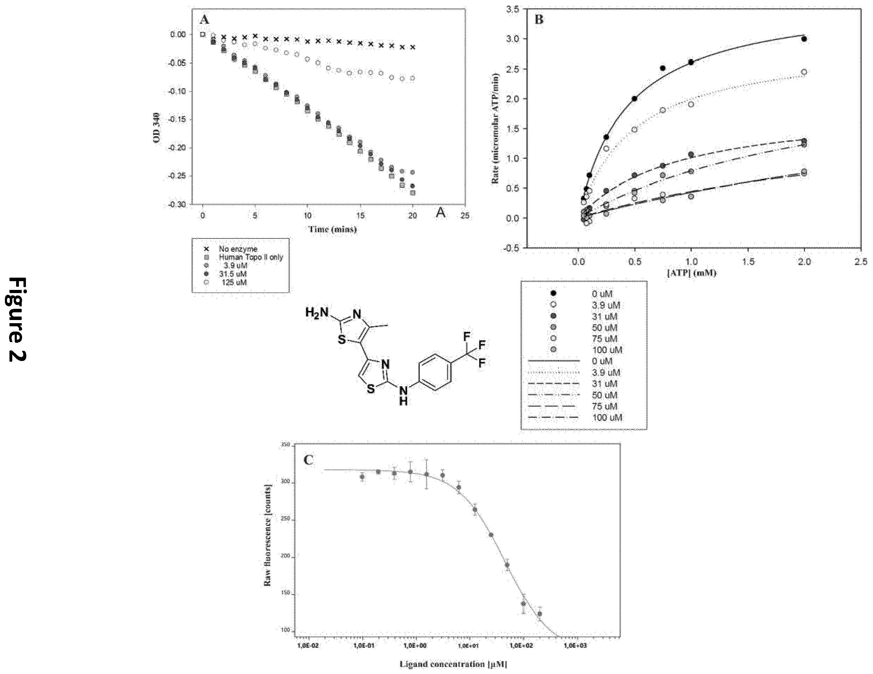

As shown in Figure 2A, compound **1** successfully inhibited 75 % (first parallel) or 85 % (second parallel, data not shown) the ATP hydrolysis at 125 µM. For comparison, etoposide inhibited 70 % (first parallel) or 63 % (second parallel) of ATPase hydrolysis at this concentration (data not shown).

Then, we performed the competitive ATPase assay for compound **1,** to investigate how our class of compounds affects the ATP hydrolysis in dependence of ATP concentration. Assay was performed at 8 different concentrations of ATP and compound **1.** In Figure 2B, the observed rates of the ATP hydrolysis are plotted against the increased ATP concentration for different concentrations of compound **1.** The assay was carried out in two parallels. From the obtained graph, it can be observed that the rate of ATP hydrolysis was significantly faster at lower concentrations of the inhibitor **1** and was subsequently showing down with the increasing concentration of the compound. This showed that **1** has a substantial concentration-dependent effect on the rate of ATP hydrolysis. In addition, we also calculated the IC₅₀ values of compound **1** vs. different concentrations of the ATP. In Table 5 these results are presented and a clear decrease in the IC₅₀ values as the ATP concentration decreases can be noticed. Such observed behaviour is in line with the envisioned ATP competitive mode of inhibition of the substituted 4,5-bithiazole class.

Since DNA topoisomerase IIα is a complex molecular motor we also studied binding of the inhibitor **1** to the isolated human topo IIα ATPase domain using a new microscale thermophoresis (MST) technique. MST is a versatile technique to characterize properties and intermolecular interactions between biomolecules and small molecules. It quantifies biomolecular interactions based on the physical principle of thermophoresis, the direct motion of molecules in the temperature gradient. The thermophoretic movement of the labelled protein in complex with selected inhibitor was measured by monitoring the fluorescence distribution inside the capillary [46-48]. MST experiments, performed in three independent runs, showed a concentration-dependent binding to the ATPase domain and yielded a K_{D} = 50.6 ± 7.6 µM for compound **1.**

These results confirmed that substituted 4,5'-bithiazoles bind to the truncated human topo IIα ATP domain, where the ATP binding site is the only available active site. This alongside and results of the competitive ATPase assay suggests the mode of inhibition via binding to the ATP binding site. Binding curve of compound 1 is represented in Figure 2C.

**Table 5. IC₅₀ values for compound 1 at different concentrations of ATP with the R² values for the generated fits.**

| | | | | | | | | | |
|---|---|---|---|---|---|---|---|---|---|
| **c(ATP)** | **2** | **1** | **0.75** | **0.5** | **0.25** | **0.1** | **0.075** | **0.05** | **0.025** |
| **IC₅₀ [**µM] | 25.4 | 21.6 | 17.8 | 15.2 | 18.3 | 9.4 | 14.8 | 8.8 | 3.8 |
| **R2** | 0.981 | 0.971 | 0.972 | 0.985 | 0.992 | 0.974 | 0.958 | 0.929 | 0.474 |

**Figure 2.** (A) Results of the ATPase assay. Compound **1** inhibited 75 % (first parallel) of human topo IIα mediated ATP hydrolysis at 125 µM. The graph shown is representative of two independent experiments. (B) The rates plotted against ATP concentration for compound **1** in competitive ATPase assay. Curves are fitted to the data using the hyperbolic equation y=(ax/(b+x) where y = rate, x = [ATP], a = Vmax and b=Km. (C) In the MST experiment we kept the concentration of human topo IIα ATPase domain labelled molecule constant (20 nM) while the concentration of the unlabelled binding partner (compound **1**) was varied between 200 and 0.0098 µM. Samples were loaded in premium capillaries (Nanotemper Technologies) and the MST measurement was performed using the Monolith NT.115 (Nanotemper Technologies). An average value (three measurements) for Kd =50.6 ± 7.6 µM was obtained for this interaction employing the initial fluorescence.

### 2. Activity of substituted 4,5'-bithiazoles on human cancer cell lines

We determined the cytotoxicity of the tested compounds on human breast cancer MCF-7 and human hepatocellular carcinoma HepG2 cancer cell lines using MTS assay [33]. Both selected human cancer cell lines are representative and well-established systems for cell-based evaluation of potential anticancer compounds. Etoposide was again used as a positive control (PC).

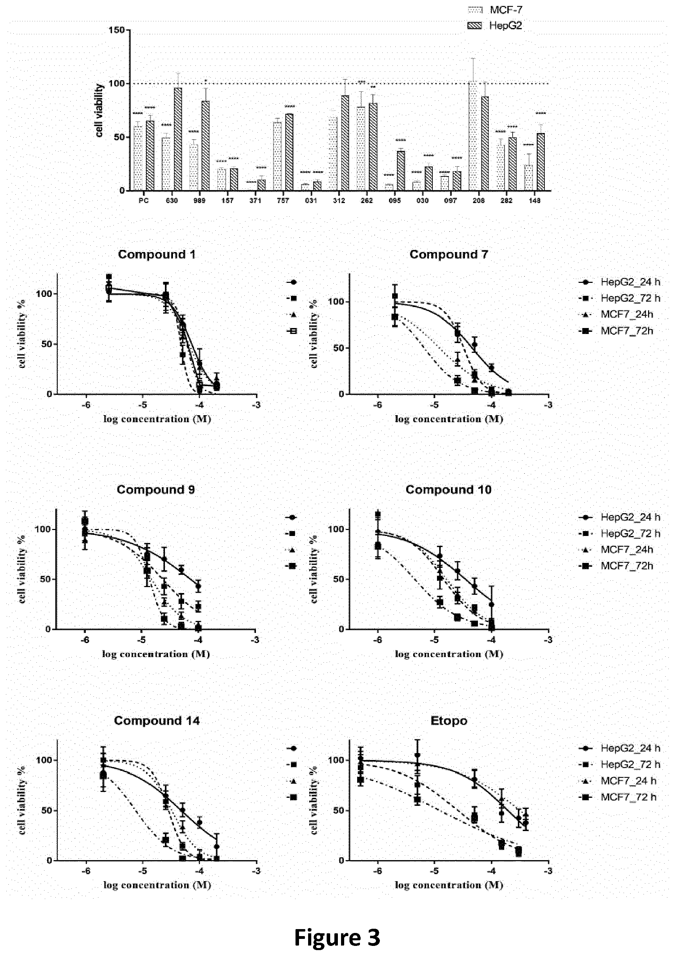

In the first step we performed the initial screening of compounds **1-14** at one concentration. Depending on the solubility of compounds in the cell growth media 200 µM was used for compounds **1,4,5,7,8,11,13,14,** 100 µM for compounds **2, 6, 9,10,12** and 50 µM for compound **3.** Exponentially growing cells were exposed to all compounds for 24 h and the results are represented in Figure 3A. For the most effective compounds the cell viability was reduced by more than 80 % relative to the untreated control on both cell lines. Further, EC₅₀ values were determined by testing their cytotoxicity at different concentrations to observe the dose-response curves for 24h (short term) and 72h (long term) exposure treatment. We chose the most potent compounds from the initial screening namely compounds **1, 7, 9, 10, 14** along with etoposide as a positive control. Unfortunately, for compound **3** we could not determine the EC₅₀ because of its low solubility in the cell growth media. Determined EC₅₀ values at 24h and 72h time interval are represented in Table 6 and obtained dose-response curves are represented in Figure 3B. For comparison, we determined the EC₅₀ values also for etoposide and confirmed that the EC₅₀ values after 72h were in the same range as for our compounds and in line with the reported data [34, 35].

Compounds were generally more cytotoxic for MCF-7 cell line in comparison to the HepG2 cells. The most potent compound was compound **10** with EC₅₀ value 4.5 µM on MCF-7 cell line after 72h treatment. To compare, compound **10** was also the most potent in the enzyme relaxation assay as an inhibitor of human topo IIα. Compounds **1, 7, 9** were also potent inhibitors of enzyme as well good cytotoxic agents with EC₅₀ values of 59.5, 6.6 and 13.8 µM, respectively, in MCF-7 cells. Compound **14** was less potent in enzyme assay (IC₅₀357.1 µM) but showed high cytotoxic activity. The reason could be a better cell permeability due to the unsubstituted phenyl substituent. Compounds **6, 8, 11** with carboxylic acid present on the phenyl ring had little or no effect on the tested cell lines. For compounds with favorable EC₅₀ after 24h treatment, we performed additional cytotoxicity measurements after 72h treatment to see long term effect on the cells. Compounds showed higher cytotoxic activity after prolonged time of exposure, which was expected. For HepG2 cells, at 72h exposure the EC₅₀ values of tested compounds were in the range between 15 and 50 µM and several compounds had superior cytotoxic activity compared to etoposide.

**Figure 3.** (A) Results of the MTS assay of substituted-4,5'-bithiazoles. HepG2 and MCF-7 cells were exposed to the investigated compounds at compounds concentration of 200 µM for compounds **1, 4, 5, 7, 8, 11, 13, 14,** 100 µM for compounds **2, 6, 9, 10, 12** and 50 µM for compound **3** for 24h. As positive control (PC), etoposide was used at 200 µM and DMSO (0.5 %) was used as solvent control. **(B)** Dose response curves for selected compounds that were titrated on the HepG2 and MCF-7 cell lines for 24h or 72h.

**Table 6. Determined values of cytotoxicity of substituted-4,5'-bithiazoles against HepG2 and MCF-7 cancer cell lines.**

| **Compound** | EC₅₀ [µM] | EC₅₀ [µM] | EC₅₀ [µM] | EC₅₀ [µM] |
|---|---|---|---|---|
| | **MCF-7** (*24h*) | **MCF-7** (*72h*) | **HepG2** (*24h*) | **HepG2** (*72h*) |
| **1** | 65.0 (58.1-70.9) | 59.5 (58.1-59.3) | 72.8 (64.2-86.1) | 46.8 (47.1-49.3) |
| **7** | 12.3 (10.4-14.9) | 6.6 (4.6-7.6) | 45.6 (32.8-53.7) | 32.3 (29.7-37.2) |
| **9** | 15.0 (11.2-16.2) | 13.8 (9.7-15.9) | 73.5 (59.6-80.8) | 23.5 (17.1-32.1) |
| **10** | 16.4 (14.6-17.0) | 4.5 (3.4-5.9) | 34.7 (31.5-38.9) | 14.6 (12.7-19.5) |
| **14** | 32.3 (28.2-34.3) | 7.5 (5.2-6.7) | 47.7 (31.7-60.4) | 28.2 (20.5-29.9) |
| **Etoposide** | 320.2 (226.9-358.9) | 12.6 (10.1-15.9) | 196.2 (169.4-201.5) | 25.8 (15.1-35.3) |

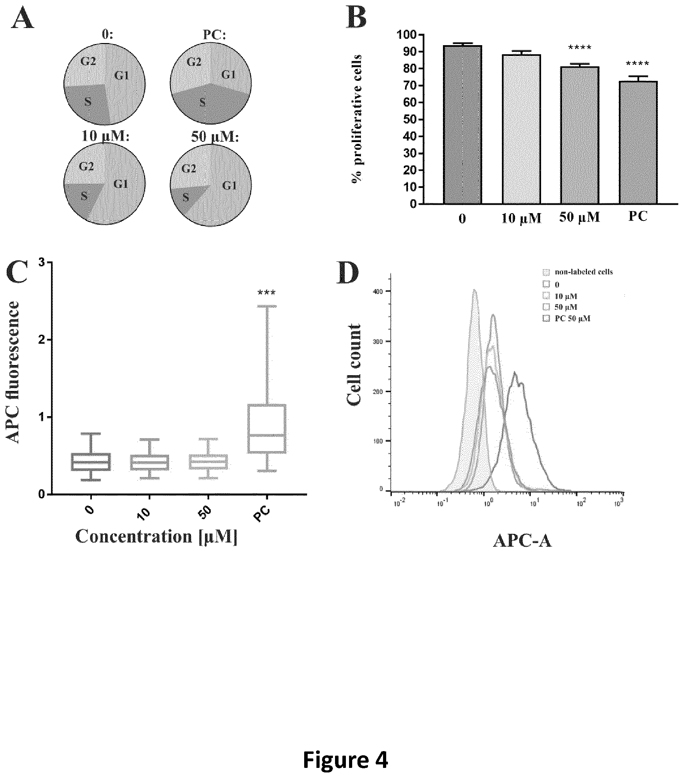

While DNA damage can, depending on when it is detected, trigger a cell cycle arrest in either G1, S or G2 phase, topoisomerase poisons seem to mainly cause the G2 phase arrest [36]. On the other hand, catalytic inhibitors do not directly damage DNA and several molecules have been reported to cause a G1 arrest [37, 38]. Consistent with this, compound **1** induced the G1-phase arrest. Compound **1** at concentration 50 µM significantly increased G1 phase of cell cycle (60.6 %) and decreased S phase (17.5 %) in comparison with solvent control (46.7 % for G1 phase and 25.9 % for S phase). In lower concentration - 10 µM, changes in cell cycle were less pronounced - 56.5 % of cells were in G1 phase and 17.5 % in S phase. Contrary, etoposide (PC) decreased G1 phase (33.1 %) and increase S phase (44.0 %) of cell cycle in comparison with the solvent control. Results of this experiment for compound **1** are presented as pie charts in Figure 4A. In the HepG2 cells treated with etoposide we observed the accumulation of the cells in the S phase of cell cycle. This is in accordance with the literature data that etoposide can induce the cell cycle arrest in the late S phase and early G2 phase [39]. By performing the cell cycle analysis, we thus confirmed that the presented compounds act via a different mechanism as topo II poisons on the cellular level.

We performed analysis of inducing Double-strand breaks (DBS) primarily to confirm also on the cellular level, besides the conformation on the in vitro level previously provided by the cleavage assay that this class of compounds does not act as topoisomerase poisons, but as catalytic inhibitors. The data also complement the conclusions from the cell cycle analysis. HepG2 cells were exposed for 24h to compound **1** and etoposide. There was no increase in the DNA DSB formation in the case of compound **1** at concentrations 10 µM and 50 µM, while etoposide, being a well-known topo II poison, significantly induced DSBs at comparable concentration of 50 µM (Figure 4C and 4D). This result is in line with the different mechanisms of action of topo IIα poisons and catalytic inhibitors at the cellular level.

**Figure 4****.** Cell cycle analysis, cell proliferation and induction of the DNA double stand breaks (DBS) by the selected substituted 4,5'-bithiazole compound **1. (A)** Percent of HepG2 cells in certain phases of cell cycle after 24h treatment to compound **1** at 50 µM and 10 µM in comparison with the solvent control (0). As positive control (PC) etoposide at 50 µM was used. **(B)** Percent of proliferative cells after 24h treatment to compound **1** at 50 µM and 10 µM determined with the Ki67 antibody. (**C**) Results of the analyses of the induction of the DNA double strand breaks by γ-H2AX assay. Distribution of the fluorescent signals of individual cells in the samples is shown. Data are presented as quantile box plots. The edges of the box represent the 25th and 75th percentiles, the median is a solid line through the box, and the bars represent 95% confidence intervals. In each sample, 10⁴ events were recorded, and experiments were repeated three times independently. Significant difference between treated cells and the solvent control (0) is indicated by * p < 0.05, ** p < 0.01 and *** p < 0.001. A solvent control (0. 5 % DMSO) and a positive control (50 µM etoposide) were included in each parallel. (**D**) Representative histograms for non-labelled cells, vehicle control (0), compound **1** at 10 µM and 50 µM and etoposide at 50 µM.

To investigate how our compound **1** effects the proliferation of HepG2 cells, we stained cells with antibodies against Ki-67 cell biomarker and analysed them with flow cytometry. The influence of compound **1** on cell proliferation inhibition was analyzed by the detection of cells positive for the proliferation marker Ki-67. The expression of the human Ki67 protein is associated with cell proliferation as the protein is present during all active phases of the cell cycle (G1, S, G2, and M), and absent from resting cells (G0) [40]. We investigated effect on cell proliferation at concentrations 50 µM and 10 µM. Our investigated compound **1** significantly decreased proliferation of HepG2 cells. Percent of cell proliferation after treatment HepG2 cells for 24h with compounds **1** was 81.3 % at concentration 50 µM. For comparison, cell proliferation after treatment of HepG2 cells for 24h with etoposide was 72.9 %. At concentration 10 µM effect on proliferation of cells was nonsignificant (88.6 %). We used one-way ANOVA for statistical analysis of results (Figure 4B).

**Table 7: Results of cell cycle analysis for compound 1. Experiments were performed in 3 independent measurements and SD values were calculated. Significant differences between the vehicle control (0) and treated cells (compound 1) as well as PC (etoposide; 50 µM) were calculated using ANOVA (**p<0.01, ***p<0.001).**

| **Compound** | **0** | **1 (10 µM)** | **1 (50 µM)** | **PC** |
|---|---|---|---|---|
| **G1** | 46.7 ± 4.6 | 56.5 ± 6.4 | 60.6 ± 7.0^{∗} | 33.1 ± 2.4^{∗} |
| **5** | 25.9 ± 3.3 | 17.5 ± 1.1^{∗∗∗} | 11.8 ± 4.5^{∗∗} | 44.0 ± 1.5^{∗∗∗} |
| **G2** | 25.4 ± 4.9 | 24.8 ± 4.5 | 26.2 ± 2.5 | 22.9 ± 3.5 |
| **>G1, G2<** | 2 ± 1.1 | 1.2 ± 1.0 | 1.4 ± 0.6 | 0 |

### Experimental Methods

### 1. HTS relaxation assay of human topo IIα

The HTS topo IIα relaxation assay of all compounds **1-15** was performed on the black streptavidin-coated 96-well microtiter plate. Plate was rehydrated using wash buffer (20 mM Tris-HCl (pH = 7.6), 137 mM NaCl, 0.005 % (w/v) BSA, 0.05 % (v/v) TWEEN-20^{®}) and biotinylated oligonucleotide was immobilized onto the wells. Next, the excess oligonucleotide was washed off with wash buffer. Enzyme assay was carried out in reaction volume of 30 µL using 0.75 µg supercoiled plasmid pNO1 as a substrate and human topo IIα. Enzyme was diluted to the appropriate concentration with dilution buffer comprised of 50 mM Tris-Hcl (pH = 7.5), 100 mM NaCl, 1 mM DTT, 0.5 mM EDTA, 50 % (v/v) glycerol and 50 µg/mL albumin. Tested compounds (0.3 µL) were added as a stock solution in DMSO and final concentration of DMSO was 1 %, respectively. Reactions were incubated at 37 °C for 30 min, and TF buffer (50 mM NaOAc (pH = 5.0), 50 mM NaCl, 50 mM MgCl₂) was added to the wells and incubated at room temperature for additional 30 min to allow the triplex biotin-oligonucleotid-plasmid formation [24]. To eliminate the aggregation and non-specific inhibition a surfactant (TWEEN-20^{®}) was added to the reaction mixture [41]. Unbound plasmid was washed off with TF buffer and stained with the DNA-detection Dye in the T10 buffer (10 mM Tris-HCl (pH = 8) and 1 mM EDTA) was added. After mixing, fluorescence was read using Tecan fluorimeter (Excitation: 485 nm and Emission: 535 nm). Screening was performed at the inhibitor concentrations of 7.8, 31.5, 125 and 500 µM. IC₅₀ values were calculated using GraphPad Prism 6.0 software [42] and represent the concentrations of inhibitors where residual activity of the enzyme was 50 % [24].

### 2. The human topo IIα cleavage assay

One U of human topo IIα was incubated with the 0.5 µg supercoiled plasmid DNA (pBR322) and the respective amount of selected active compounds **1** and **9** or reference compound etoposide in a 30 µL reaction at 37 °C for 60 minutes under the following conditions: 20 mM Tris HCl (pH 7.5), 200 mM NaCl, 0.25 mM EDTA and 5 % glycerol. Selected compounds were tested at four concentrations: 3.9, 31.5, 125 and 500 µM. The reaction was then incubated for additional 30 minutes with 0.2 % SDS and 0.5 µg/µL proteinase K. Subsequently the reaction was stopped by adding 30 µL of chloroform/iso-amyl alcohol (26:1) and 30 µL of Stop Dye (40 % sucrose (w/v), 100 mM Tris.HCl (pH 7.5), 10 mM EDTA, 0.5 µg/mL bromophenol blue), before sample being loaded on the 1 % TAE gel run at 80 V for 2 hours.

Bands were visualized by the ethidium bromide staining for 15 minutes and destaining for subsequent 10 minutes. Gels were scanned using the documentation equipment (GeneGenius, Syngene, Cambridge, UK) and cleavage levels were calculated from the band data obtained by the gel scanning software (GeneTools, Syngene, Cambridge,UK). The human topo IIα cleavage assay was performed in collaboration with Inspiralis (Norwich, UK).

### 3. The human topo IIα and human topo IIβ decatenation assay

The human topo II decatenation assay kit from Inspiralis (Norwich, UK) was used to determine if compounds inhibit the DNA decatenation [43, 44]. One U of human topo II (α or β) was incubated with 200 ng kDNA in a 30 µL reaction at 37°C for 30 minutes under the following conditions: 50 mM Tris HCl (pH 7.5), 125 mM NaCl, 10 mM MgCl2, 5 mM DTT, 0.5 mM EDTA, 0.1 mg/ml bovine serum albumin (BSA) and 1 mM ATP. Compounds **1** and **9** were serially diluted in DMSO and added to the reaction mixture before the addition of the enzyme. Etoposide was used as a control compound. Each reaction was then stopped by the addition of 30 µL chloroform/iso-amyl alcohol (26:1) and 30 µL Stop Dye (40 % sucrose (w/v), 100 mM Tris.HCl (pH 7.5), 10 mM EDTA, 0.5 µg/mL bromophenol blue), before being loaded on a 1% TAE gel run at 85 V for 90 minutes. Assays were performed at 4 concentrations of ligand: 3.9, 31.5, 125 and 500 µM. The final DMSO concentration in all reactions was 1 % (v/v).

Bands were visualized by the ethidium bromide staining for 15 minutes and destaining for subsequent 10 minutes. Gels were scanned using the documentation equipment (GeneGenius, Syngene, Cambridge, UK) and inhibition levels were calculated from band data obtained with gel scanning software (GeneTools, Syngene, Cambridge,UK). Experiment was performed in duplicates for all compounds.

### 4. Inhibition of ATPase activity

Compounds were analyzed using a linked assay in which the ADP produced by the hydrolysis of ATP leads to conversion of NADH to NAD by a pyruvate kinase/lactate dehydrogenase mix. The disappearance of NADH is monitored at 340 nm. A mix of assay buffer (5 µL of 10X buffer per assay: final conc. 20 mM Tris-HCl, 5 mM magnesium acetate, 125 mM potassium acetate, 2 mM DTT, pH 7.9), linear pBR322 (1.5 µL of 1mg/mL per assay), phosphoenol pyruvate (0.5 µL of 80 mM per assay), pyruvate kinase/lactate dehydrogenase mix (0.75 µL per assay), NADH (1 µL of 20 mM per assay) and water (34.35 µL per assay) was made. 41.1 µL was aliquoted into the wells of a 384-well microtitre plate. 0.5 µL of DMSO, etoposide or the appropriate compound were added to the wells and mixed. 5 µL of the dilution buffer or human topo IIα (200 nM stock giving 12 nM final concentration) were then added and mixed. The change in OD340 was then measured in a plate reader over a period of about 10 minutes (called the prerun). 3.4 µL of 30 mM ATP was then added and the OD340 monitored for up to 30 minutes. Assays were performed at 37 °C. The final DMSO concentration in all the reactions was 1 % (v/v). Assays were performed at 3.9, 31.5, 125, and 500 µM final concentrations of the investigated compound **1.** Compound were serially diluted in DMSO and added to the reaction before the addition of the enzyme. Control compound for was Etoposide. Determination if compound **1** from class of substituted 4,5'-bithiazoles inhibits ATPase activity of human topo II enzyme was performed in collaboration with Inspiralis.

### 5. Human topoisomerase II ATPase assays at different concentration of ATP - competitive ATPase assay

Selected active compound **1** were dissolved and diluted in DMSO and added to the reaction before the addition of the enzyme. Compound were analyzed using a linked ATPase assay in which the ADP produced by the hydrolysis of ATP leads to the conversion of NADH to NAD by a pyruvate kinase/lactate dehydrogenase mix. The disappearance of NADH was monitored at 340 nm [33]. A mix of the assay buffer (5 µL of 10X buffer per assay: final conc. 20 mM Tris-HCl, 5 mM magnesium acetate, 125 mM potassium acetate, 2 mM DTT, pH 7.9), linear pBR322 (1.5 µL of 1mg/mL per assay), phosphoenol pyruvate (0.5 µL of 80 mM per assay), pyruvate kinase/lactate dehydrogenase mix (0.75 µL per assay), NADH (1 µL of 20 mM per assay), DMSO (1.5 µL per assay), and water (32.85 µL per assay) was made. 41.1 µL of this mixture was aliquoted into the wells of a 384-well microtiter plate. 0.5 µL of DMSO or the investigated compound were added to the wells and mixed. 5 µL of the dilution buffer or human topo IIα (200 nM stock giving the 12 nM final concentration) were then added and mixed. A pre-run was done before the run was started by adding 3.4 µL of the appropriate concentration of ATP and the OD340 monitored for up to 35 minutes. Assays were performed at 37 °C. Two negative controls (4 % DMSO and dilution buffer without enzyme) were run in the presence of 2 mM ATP. This assay was performed at 8 different concentrations of the ATP (0.025, 0.05, 0.075, 0.1, 0.25, 0.5, 0.75 and 1 mM). Assays were performed at 3.9, 31, 50, 75 and 100 µM final concentrations of the investigated compound **1.** The final DMSO concentration in all the reactions was 4 % (v/v). Assay was performed in duplicate. The human topo competitive ATPase assay was performed in collaboration with Inspiralis (Norwich, UK).

### 6. Microscale thermophoresis (MST) measurements of compound 1 binding onto human topo IIα ATPase domain

MST measurements were performed on a Monolith NT 115 (NanoTemper Technologies, München, Germany) using LED 20 % and MST power of 20 %. The protein - The ATPase fragment with 1-453 amino acid residues was purchase from Inspiralis [45]. It was labeled with NT-647 dye using Nanotemper Protein Labeling Kit RED-MALEIMIDE (Cysteine Reactive; no. L004, NanoTemper Technologies) according to the supplied protocol in the supplied labelling buffer applying a concentration of 20 µM protein (molar dye : protein ration = 1:3) at room temperature for 30 minutes. Next, unreached dye was removed prior to binding analysis by a gravity flow column and concurrently rebuffered in the MST buffer (50 mM Tris-HCl (pH = 7.4), 150 mM NaCl, 10 mM MgCl₂, 0.05 % Tween-20) [46-48]. The concentration of the labelled protein was kept constant at ~ 20 nM concentrations with MST buffer. A twofold dilution series were prepared for the unlabelled compound with the final concentrations ranging from 0.098 µM to 200 µM (12 concentrations). Samples were prepared and measured three times to calculate average Kd values and SD. DMSO concentrations at 10 % was constant sample to sample. The samples were loaded in premium capillaries (MO-K025, NanoTemper Technologies, München, Germany). Thermophoresis was measured using a Monolith NT 115 (NanoTemper Technologies, Munich, Germany) at an ambient temperature 25 °C with 5 s/30 s/5 s laser off/on/off times, respectively. Because of titrant dependent fluorescence changes in the first step of measuring, SDS-denaturation test was performed to confirm specific, ligand-induced binding and data measurements was analyzed (MO, Affinity Analysis, NanoTemper Technology) using the signal from initial fluorescence.

### 7. In vitro cytotoxicity measurements using MTS assay on MCF-7 and HepG2 cell lines

*In vitro* cytotoxicity of investigated compounds was evaluated in two human cancer cell lines: the breast cancer HepG2 and hepatocellular carcinoma MCF7-7 cell lines. The HepG2 cells (ATCC) were cultivated at 37 °C and 5 % CO₂ in MEME medium (M2414, Sigma) supplemented with 10 % FBS, 2 mM L-Glutamine, 1 % NEAA, 2.2 g/L NaHCO3, 1 mM sodium pyruvate and 100 IU/mL penicillin/ streptomycin and MCF-7 (ATCC) were cultivated in Eagle's MEM medium (M5650, Sigma), supplemented with 10 % fetal bovine serum, 2 mM glutamine, and 100 IU/mL penicillin/streptomycin. After the exposure to the tested compounds the viability of HepG2 and MCF-7 cells was determined by MTS tetrazolium reduction assay. The cells were seeded onto 96-well microplates (Nunc, TermoFisher Scientific, Waltham, MA, USA) at densities of 8000 cells per well (HepG2) and 7000 cells per well (MCF-7) in 200 mL growth medium and incubated overnight to attach. The next day, the growth medium was replaced by the fresh complete growth medium containing appropriate concentrations of the compounds. Prepared microplates were then incubated for additional 24 h and afterwards 40 µL of freshly prepared MTS : PMS solution (20:1) was added directly to the 200 µL of medium in the culture wells and incubated for additional 3 h (37 °C, 5 % CO₂). Finally, the absorbance was measured at wavelength of 490 nm using a Microplate Reader (Synergy MX, BioTek, Winooski, VT, USA). As positive control, etoposide at 200 µM was used. Assay was performed at 5 concentrations of each compound; 2, 25, 50, 100 and 200 µM for compounds **1, 7** and **14** and 1, 12.5, 25, 50, 100 µM for compounds **9** and **10.** In addition, etoposide was titrated at 5, 50, 100, 150 and 300 µM. Cell viability was determined by comparing the OD (optical density) of the wells containing the cells treated with investigated compounds with those of solvent control cells and presented as % of cell viability ± SD. Experiments were performed three times independently each time in at least three replicates. EC₅₀ values were calculated by non-linear regression analysis using GraphPad Prism 7.0 Software. Statistical significance between treated groups and the control was determined by One-way analysis of variance and Dunnett's Multiple Comparison Test.

### 8. Effect of selected compounds on the cell cycle, cell proliferation and on induction of the DNA double stranded breaks

The HepG2 cells were seeded on the 25 cm² plates (Corning Inc., NY, USA) (750 000 cells/plate), left to attach overnight and exposed to the selected compound **1** (10 µM and 50 µM) and etoposide (positive control, 50 µM) for 24 h. After the exposure floating and adherent cells were collected by trypsinization. For the fixation the cells were centrifuged at 800 rpm, 4°C for 5 min, washed twice with ice cold PBS, resuspended in 0.5 mL cold PBS and ethanol (1.5 mL) was added drop wise into the cell pellet, while vortexing. The cells were fixed at 4°C overnight and stored at -20°C until analysis. Fixed cells were centrifuged at 1200 rpm for 10 min, washed with ice cold 1× PBS and labelled with Anti-H2AX pS139 antibodies (50-fold diluted) for DNA DBS analysis, Ki67 antibodies (50-fold diluted) for proliferation analysis and Hoechst 33342 dye for cell cycle analysis according to the manufacturer's protocol. Flow cytometric analysis was carried out on a MACSQuant Analyzer 10 (Miltenyi Biotech, Germany). FITC intensity, corresponding to proliferation marker Ki67+, was detected in the FITC-A channel and cell cycle analysis was detected in VioBlueA channel. APC intensity, corresponding to DSBs, was detected in the APC-A channel. Unspecific binding was checked with rea-FITC and rea-APC antibodies (Miltenyi Biotec, Germany). In each sample, 10 000 events were recorded. Independent experiments were repeated three times. In all experiments, 50 µM etoposide was included as positive control and 0.5 % DMSO as vehicle control. For further analysis the raw data was exported from MACSQuantify software and it was converted to. fcs format and further to the .csv format. For the yH2AX foci analysis the statistical significance between treated groups and the vehicle control was determined with a linear mixed-effects model. Calculations were done with the statistical program R [49] and its packages reshape [50] and nlme [51].

### REFERENCES

[1] J.C. Wang, Cellular roles of DNA topoisomerases: A molecular perspective, Nature Reviews Molecular Cell Biology, 3 (2002) 430-440.
[2] J.J. Champoux, DNA topoisomerases: Structure, function, and mechanism, Annual Review of Biochemistry, 70 (2001) 369-413.
[3] J.L. Nitiss, DNA topoisomerase II and its growing repertoire of biological functions, Nature Reviews Cancer, 9 (2009) 327-337.
[4] F.H. Drake, J.P. Zimmerman, F.L. McCabe, H.F. Bartus, S.R. Per, D.M. Sullivan, W.E. Ross, M.R. Mattern, R.K. Johnson, S.T. Crooke, et al., Purification of topoisomerase II from amsacrine-resistant P388 leukemia cells. Evidence for two forms of the enzyme, J. Biol. Chem., 262 (1987) 16739-16747.
[5] G. Capranico, S. Tinelli, C.A. Austin, M.L. Fisher, F. Zunino, Different patterns of gene expression of topoisomerase II isoforms in differentiated tissues during murine development, Biochim. Biophys. Acta, 1132 (1992) 43-48.
[6] M. Watanabe, K. Tsutsui, K. Tsutsui, Y. Inoue, Differential expressions of the topoisomerase II alpha and II beta mRNAs in developing rat brain, Neurosci. Res., 19 (1994) 51-57.
[7] K. Tsutsui, K. Tsutsui, O. Hosoya, K. Sano, A. Tokunaga, Immunohistochemical analyses of DNA topoisomerase II isoforms in developing rat cerebellum, J. Comp. Neurol., 431 (2001) 228-239.
[8] J.L. Delgado, C.M. Hsieh, N.L. Chan, H. Hiasa, Topoisomerases as anticancer targets, Biochem J, 475 (2018) 373-398.
[9] B. Pogorelčnik, A. Perdih, T. Solmajer, Recent Developments of DNA Poisons - Human DNA Topoisomerase II alpha Inhibitors - as Anticancer Agents, Current Pharmaceutical Design, 19 (2013) 2474-2488.
[10] W. Hu, X.S. Huang, J.F. Wu, L. Yang, Y.T. Zheng, Y.M. Shen, Z.Y. Li, X. Li, Discovery of Novel Topoisomerase II Inhibitors by Medicinal Chemistry Approaches, J Med Chem, 61 (2018) 8947-8980.
[11] C. Bailly, Contemporary challenges in the design of topoisomerase II inhibitors for cancer chemotherapy, Chemical reviews, 112 (2012) 3611-3640.
[12] G. Minotti, P. Menna, E. Salvatorelli, G. Cairo, L. Gianni, Anthracyclines: Molecular advances and pharmacologic developments in antitumor activity and cardiotoxicity, Pharmacological Reviews, 56 (2004) 185-229.
[13] C.A. Felix, Secondary leukemias induced by topoisomerase-targeted drugs, Biochim Biophys Acta, 1400 (1998) 233-255.
[14] G. Housman, S. Byler, S. Heerboth, K. Lapinska, M. Longacre, N. Snyder, S. Sarkar, Drug resistance in cancer: an overview, Cancers (Basel), 6 (2014) 1769-1792.
[15] P. Pilati, D. Nitti, S. Mocellin, Cancer resistance to type II topoisomerase inhibitors, Current medicinal chemistry, 19 (2012) 3900-3906.
[16] G. Szakács, J.K. Paterson, J.A. Ludwig, C. Booth-Genthe, M.M. Gottesman, Targeting multidrug resistance in cancer, Nature Reviews Drug Discovery, 5 (2006) 219-234.
[17] R.N. Ganapathi, M.K. Ganapathi, Mechanisms regulating resistance to inhibitors of topoisomerase II, Front Pharmacol, 4 (2013) 89-89.
[18] E.L. Baldwin, N. Osheroff, Etoposide, topoisomerase II and cancer, Curr Med Chem Anticancer Agents, 5 (2005) 363-372.
[19] E.M. Nelson, K.M. Tewey, L.F. Liu, Mechanism of Antitumor Drug-Action - Poisoning of Mammalian DNA Topoisomerase-li on DNA by 4'-(9-Acridinylamino)-Methanesulfon-Meta-Anisidide, Proceedings of the National Academy of Sciences of the United States of America-Biological Sciences, 81 (1984) 1361-1365.
[20] B. Pogorelčnik, A. Perdih, T. Solmajer, Recent Advances in the Development of Catalytic Inhibitors of Human DNA Topoisomerase II alpha As Novel Anticancer Agents, Current medicinal chemistry, 20 (2013) 694-709.
[21] K. Bergant, M. Janezic, K. Valjavec, I. Sosic, S. Pajk, M. Stampar, B. Zegura, S. Gobec, M. Filipic, A. Perdih, Structure-guided optimization of 4,6-substituted-1,3,5-triazin-2(1H)-ones as catalytic inhibitors of human DNA topoisomerase Ilalpha, European journal of medicinal chemistry, 175 (2019) 330-348.
[22] P. Chene, J. Rudloff, J. Schoepfer, P. Furet, P. Meier, Z. Qian, J.M. Schlaeppi, R. Schmitz, T. Radimerski, Catalytic inhibition of topoisomerase II by a novel rationally designed ATP-competitive purine analogue, BMC Chem Biol, 9 (2009) 1.
[23] L.H. Jensen, A.V. Thougaard, M. Grauslund, B. Sokilde, E.V. Carstensen, H.K. Dvinge, D.A. Scudiero, P.B. Jensen, R.H. Shoemaker, M. Sehested, Substituted purine analogues define a novel structural class of catalytic topoisomerase II inhibitors, Cancer Research, 65 (2005) 7470-7477.
[24] A. Maxwell, N.P. Burton, N. O'Hagan, High-throughput assays for DNA gyrase and other topoisomerases, Nucleic Acids Res, 34 (2006).
[25] G.L. Chen, L. Yang, T.C. Rowe, B.D. Halligan, K.M. Tewey, L.F. Liu, Nonintercalative Antitumor Drugs Interfere with the Breakage-Reunion Reaction of Mammalian DNA Topoisomerase-li, Journal of Biological Chemistry, 259 (1984) 3560-3566.
[26] W.B. Wu, J.B. Ou, Z.H. Huang, S.B. Chen, T.M. Ou, J.H. Tan, D. Li, L.L. Shen, S.L. Huang, L.Q. Gu, Z.S. Huang, Synthesis and evaluation of mansonone F derivatives as topoisomerase inhibitors, European journal of medicinal chemistry, 46 (2011) 3339-3347.
[27] A.M. Azarova, Y.L. Lyu, C.P. Lin, Y.C. Tsai, J.Y. Lau, J.C. Wang, L.F. Liu, Roles of DNA topoisomerase II isozymes in chemotherapy and secondary malignancies, Proc. Natl. Acad. Sci. U. S. A., 104 (2007) 11014-11019.
[28] Y.L. Lyu, J.E. Kerrigan, C.P. Lin, A.M. Azarova, Y.C. Tsai, Y. Ban, L.F. Liu, Topoisomerase Ilbeta mediated DNA double-strand breaks: implications in doxorubicin cardiotoxicity and prevention by dexrazoxane, Cancer Res, 67 (2007) 8839-8846.
[29] N. Akimitsu, N. Adachi, H. Hirai, M.S. Hossain, H. Hamamoto, M. Kobayashi, Y. Aratani, H. Koyama, K. Sekimizu, Enforced cytokinesis without complete nuclear division in embryonic cells depleting the activity of DNA topoisomerase Ilalpha, Genes Cells, 8 (2003) 393-402.
[30] X. Yang, W. Li, E.D. Prescott, S.J. Burden, J.C. Wang, DNA topoisomerase Ilbeta and neural development, Science, 287 (2000) 131-134.
[31] A. Sakaguchi, A. Kikuchi, Functional compatibility between isoform alpha and beta of type II DNA topoisomerase, J Cell Sci, 117 (2004) 1047-1054.
[32] S. Kenig, V. Faoro, E. Bourkoula, N. Podergajs, T. lus, M. Vindigni, M. Skrap, T. Lah, D. Cesselli, P. Storici, A. Vindigni, Topoisomerase Ilbeta mediates the resistance of glioblastoma stem cells to replication stress-inducing drugs, Cancer Cell Int, 16 (2016) 58.
[33] K. Bergant, M. Janezic, A. Perdih, Bioassays and In Silico Methods in the Identification of Human DNA Topoisomerase Ilalpha Inhibitors, Current medicinal chemistry, 25 (2018) 3286-3318.
[34] A. Alpsoy, S. Yasa, U. Gunduz, Etoposide resistance in MCF-7 breast cancer cell line is marked by multiple mechanisms, Biomedicine & pharmacotherapy = Biomedecine & pharmacotherapie, 68 (2014) 351-355.
[35] B.S. Xie, H.C. Zhao, S.K. Yao, D.X. Zhuo, B. Jin, D.C. Lv, C.L. Wu, D.L. Ma, C. Gao, X.M. Shu, Z.L. Ai, Autophagy inhibition enhances etoposide-induced cell death in human hepatoma G2 cells, International journal of molecular medicine, 27 (2011) 599-606.
[36] G.K. Dasika, S.C. Lin, S. Zhao, P. Sung, A. Tomkinson, E.Y. Lee, DNA damage-induced cell cycle checkpoints and DNA strand break repair in development and tumorigenesis, Oncogene, 18 (1999) 7883-7899.
[37] Darpan, G. Joshi, S.M. Amrutkar, A.T. Baviskar, H. Kler, S. Singh, U.C. Banerjee, R. Kumar, Synthesis and biological evaluation of new 2,5-dimethylthiophene/furan based N-acetyl pyrazolines as selective topoisomerase II inhibitors, RSC Advances, 6 (2016) 14880-14892.
[38] M.S. Islam, S. Park, C. Song, A.A. Kadi, Y. Kwon, A.F. Rahman, Fluorescein hydrazones: A series of novel non-intercalative topoisomerase Ilalpha catalytic inhibitors induce G1 arrest and apoptosis in breast and colon cancer cells, European journal of medicinal chemistry, 125 (2017) 49-67.
[39] J.M. Henwood, R.N. Brogden, Etoposide. A Review of its Pharmacodynamic and Pharmacokinetic Properties, and Therapeutic Potential in Combination Chemotherapy of Cancer, Drugs, 39 (1990) 438-490.
[40] S. Bruno, Z. Darzynkiewicz, Cell cycle dependent expression and stability of the nuclear protein detected by Ki-67 antibody in HL-60 cells, Cell proliferation, 25 (1992) 31-40.
[41] A.J. Ryan, N.M. Gray, P.N. Lowe, C.W. Chung, Effect of detergent on "promiscuous" inhibitors, Journal of Medicinal Chemistry, 46 (2003) 3448-3451.
[42] L.J.C.U. GraphPad Software, in.
[43] T.R. Hammonds, A. Maxwell, The DNA dependence of the ATPase activity of human DNA topoisomerase Ilalpha, J Biol Chem, 272 (1997) 32696-32703.
[44] Osheroff Neil, B. Mary-Ann, DNA Topoisomerase Protocols 2000.
[45] S. Campbell, A. Maxwell, The ATP-operated clamp of human DNA topoisomerase Ilalpha: hyperstimulation of ATPase by "piggy-back" binding, Journal of Molecular Biology, 320 (2002) 171-188.
[46] M. Jerabek-Willemsen, C.J. Wienken, D. Braun, P. Baaske, S. Duhr, Molecular Interaction Studies Using Microscale Thermophoresis, Assay and Drug Development Technologies, 9 (2011) 342-353.
[47] S.A.I. Seidel, P.M. Dijkman, W.A. Lea, G. van den Bogaart, M. Jerabek-Willemsen, A. Lazic, J.S. Joseph, P. Srinivasan, P. Baaske, A. Simeonov, I. Katritch, F.A. Melo, J.E. Ladbury, G. Schreiber, A. Watts, D. Braun, S. Duhr, Microscale thermophoresis quantifies biomolecular interactions under previously challenging conditions, Methods, 59 (2013) 301-315.
[48] C.J. Wienken, P. Baaske, U. Rothbauer, D. Braun, S. Duhr, Protein-binding assays in biological liquids using microscale thermophoresis, Nature Communications, 1 (2010).
[49] R.D.C. Team, A Language and Environment for Statistical Computing, in, R Foundation for Statistical Computing, Vienna, Austria, 2012.
[50] H. Wickham, Reshaping Data with the reshape Package, 2007, 21 (2007) 20.
[51] H. Wickham, ggplot2: Elegant Graphics for Data Analysis, Springer Publishing Company, Incorporated, 2009.
[52] Piala A. T. et al.: "Discovery of novel TAOK2 inhibitor scaffolds from high-throughput screening.", BIOORGANIC AND MEDICINAL CHEMISTRY LETTERS, vol. 26,6July 2016(2016-07-06), pages 3923-3927.
[53] Mori M. et al.: "A Combination Strategy to Inhibit Pim-1: Synergism between Noncompetitive and ATP-Competitive Inhibitors", CHEMMEDCHEM, vol. 8, no. 3, 22 February 2013(2013-02-22), pages 484-496

## Claims

1. A compound of general formula (I), or a pharmaceutically acceptable salt, solvate, amide, ester, ether, or N-oxide thereof, 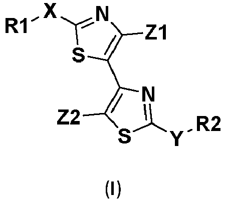
for use in combination with another anticancer agent in the treatment of a cancer;
wherein
R1 is hydrogen, -C(O)R1', substituted or unsubstituted C₁₋₆ alkyl, substituted or unsubstituted C₂₋₆ alkenyl, substituted or unsubstituted C₂₋₆ alkynyl, substituted or unsubstituted cycloalkyl, substituted or unsubstituted cycloalkenyl, substituted or unsubstituted aryl, or substituted or unsubstituted heteroaryl;
R1' is -OH, substituted or unsubstituted C₁₋₆ alkyl, substituted or unsubstituted C₂₋₆ alkenyl, substituted or unsubstituted C₂₋₆ alkynyl, substituted or unsubstituted cycloalkyl, substituted or unsubstituted cycloalkenyl, substituted or unsubstituted aryl, or substituted or unsubstituted heteroaryl;
R2 is substituted or unsubstituted aryl, or substituted or unsubstituted heteroaryl;
X is -O-, -S-, -CH₂-, or -NH-;
Y is -O-, -S-, -CH₂-, or -NH-;
Z1 is hydrogen, substituted or unsubstituted C₁₋₆ alkyl, substituted or unsubstituted C₂₋₆ alkenyl, substituted or unsubstituted C₂₋₆ alkynyl, substituted or unsubstituted cycloalkyl, substituted or unsubstituted cycloalkenyl, substituted or unsubstituted aryl, or substituted or unsubstituted heteroaryl; and
Z2 is hydrogen, substituted or unsubstituted C₁₋₆ alkyl, substituted or unsubstituted C₂₋₆ alkenyl, substituted or unsubstituted C₂₋₆ alkynyl, substituted or unsubstituted cycloalkyl, substituted or unsubstituted cycloalkenyl, substituted or unsubstituted aryl, or substituted or unsubstituted heteroaryl;
wherein the anticancer agent is selected from the group consisting of type II topoisomerase inhibitors including topoisomerase II poisons and catalytic inhibitors of topoisomerase II, type I topoisomerase inhibitors, Hsp90 inhibitors, proteasome inhibitors, inhibitors of histone deacetylases, protein kinase inhibitors, telomerase inhibitors and microtubule-interfering agents.

2. Compound for use according to claim 1, wherein R2 is a substituted or unsubstituted monocyclic aryl or substituted or unsubstituted monocyclic heteroaryl, preferably R2 is a substituted or unsubstituted monocyclic aryl, more preferably R2 is a substituted or unsubstituted phenyl.

3. Compound for use according to claim 1, wherein the compound of formula (I) is a compound of formula (II) 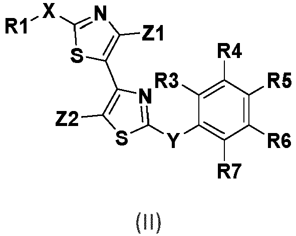 wherein
R1 is hydrogen, -C(O)R1', substituted or unsubstituted C₁₋₆ alkyl, substituted or unsubstituted C₂₋₆ alkenyl, substituted or unsubstituted C₂₋₆ alkynyl, substituted or unsubstituted cycloalkyl, substituted or unsubstituted cycloalkenyl, substituted or unsubstituted substituted or unsubstituted aryl, or substituted or unsubstituted heteroaryl;
R1' is -OH, substituted or unsubstituted C₁₋₆ alkyl, substituted or unsubstituted C₂₋₆ alkenyl, substituted or unsubstituted C₂₋₆ alkynyl, substituted or unsubstituted cycloalkyl, substituted or unsubstituted cycloalkenyl, substituted or unsubstituted substituted or unsubstituted aryl, or substituted or unsubstituted heteroaryl;
R3, R4, R5, R6 and R7 are independently selected from the group consisting of hydrogen, hydroxyl, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic heteroaryl, substituted or unsubstituted C₁₋₆ alkoxy, -C(O)R8, - S(O)2NR9R9', and -NHCOR10;
R8 is -OH or substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6-membered heteroaryl, preferably is -OH or substituted or unsubstituted C₁₋₆ alkyl;
R9 is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6-membered heteroaryl, preferably is substituted or unsubstituted oxazolyl;
R9' is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6-membered heteroaryl, preferably is hydrogen;
R10 is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6-membered heteroaryl, preferably is -CH_{3;}
X is -O-, -S-, -CH₂-, or -NH-, preferably X is -NH-;
Y is -O-, —S—, -CH₂-, or -NH-, preferably Y is -NH-; and
Z1 is hydrogen, substituted or unsubstituted C₁₋₆ alkyl, substituted or unsubstituted C₂₋₆ alkenyl, substituted or unsubstituted C₂₋₆ alkynyl, substituted or unsubstituted cycloalkyl, substituted or unsubstituted cycloalkenyl, substituted or unsubstituted aryl, or substituted or unsubstituted heteroaryl; and
Z2 is hydrogen, substituted or unsubstituted C₁₋₆ alkyl, substituted or unsubstituted C₂₋₆ alkenyl, substituted or unsubstituted C₂₋₆ alkynyl, substituted or unsubstituted cycloalkyl, substituted or unsubstituted cycloalkenyl, substituted or unsubstituted aryl, or substituted or unsubstituted heteroaryl.

4. Compound for use according to any one of claims 1 to 3, wherein Z1 and Z2 are each independently hydrogen or substituted or unsubstituted C₁₋₆ alkyl.

5. Compound for use according to claim 1, wherein the compound of formula (I) is a compound of formula (III) 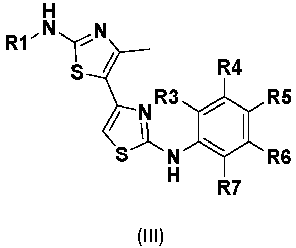 wherein
R1 is hydrogen, -C(O)R1', substituted or unsubstituted C₁₋₆ alkyl, substituted or unsubstituted C₂₋₆ alkenyl, substituted or unsubstituted C₂₋₆ alkynyl, substituted or unsubstituted cycloalkyl, substituted or unsubstituted cycloalkenyl, substituted or unsubstituted substituted or unsubstituted aryl, or substituted or unsubstituted heteroaryl;
R1' is -OH, substituted or unsubstituted C₁₋₆ alkyl, substituted or unsubstituted C₂₋₆ alkenyl, substituted or unsubstituted C₂₋₆ alkynyl, substituted or unsubstituted cycloalkyl, substituted or unsubstituted cycloalkenyl, substituted or unsubstituted substituted or unsubstituted aryl, or substituted or unsubstituted heteroaryl;
R3, R4, R5, R6 and R7 are independently selected from the group consisting of hydrogen, hydroxyl, substituted or unsubstituted C₁₋₆ alkyl, substituted or unsubstituted C₂₋₆alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic heteroaryl, substituted or unsubstituted C₁₋₆ alkoxy, -C(O)R8, -S(O)₂NR9R9', and -NHCOR10;
R8 is -OH or substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6-membered heteroaryl, preferably is -OH or substituted or unsubstituted C₁₋₆ alkyl;
R9 is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6-membered heteroaryl, preferably is substituted or unsubstituted oxazolyl;
R9' is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6-membered heteroaryl, preferably is hydrogen; and
R10 is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6-membered heteroaryl, preferably is-CH₃.

6. Compound for use according any one of claims 3 to 5, wherein R5 is selected from the group consisting of hydrogen, hydroxyl, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic heteroaryl, substituted or unsubstituted C₁₋₆ alkoxy, -C(O)R8, - S(O)₂NR9R9', and -NHCOR10;
R8 is -OH or substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6-membered heteroaryl, preferably is -OH or substituted or unsubstituted C₁₋₆ alkyl;
R9 is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6-membered heteroaryl, preferably is substituted or unsubstituted oxazolyl; and
R9' is hydrogen, substituted or unsubstituted C₁₋₆ alkyl, substituted or unsubstituted C₂₋₆ alkenyl, substituted or unsubstituted C₂₋₆ alkynyl, substituted or unsubstituted monocyclic cycloalkyl, substituted or unsubstituted monocyclic cycloalkenyl, substituted or unsubstituted monocyclic aryl, or substituted or unsubstituted monocyclic 5- or 6-membered heteroaryl, preferably is hydrogen.

7. Compound for use according to any one of claims 3 to 6, wherein R5 is selected from the group consisting of -H, -OH, -CH₃, -CF₃, -COOH, -COCH₃, -SO₂NH₂, -OCH₃, -NHCOCH₃, and 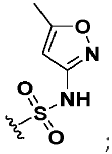 and/or
wherein R3, R4, R6 and R7 are independently selected from the group consisting of -H, - OH, -CH₃, -CF₃, -COOH, -COCH₃, -SO₂NH₂, -OCH₃, and -NHCOCH₃.

8. Compound for use according to any one of claims 3 to 7, wherein R3, R4, R6 and R7 are independently selected from the group consisting of -H, -OH, -CH₃, -CF₃, -COOH, -COCH₃, -SO₂NH₂, -OCH₃, and -NHCOCH₃.

9. Compound for use according to any one of claims 3 to 8, wherein R3 and R4 are each -H; wherein R6 is -H, -OH, or -COOH; and/or wherein R7 is -H or -CH₃.

10. Compound for use according to any one of claims 1 to 9, wherein said compound is selected from the group consisting of: 4'-methyl-N2-(4-(trifluoromethyl)phenyl)-[4,5'-bithiazole]-2,2'-diamine, 4-((2'-amino-4'-methyl-[4,5'-bithiazol]-2-yl)amino)-3-methylphenol, 1-(4-((2'-amino-4'-methyl-[4,5'-bithiazol]-2-yl)amino)phenyl)ethan-1-one, 4-((2'-amino-4'-methyl-[4,5'-bithiazol]-2-yl)amino)-N-(5-methylisoxazol-3-yl)benzenesulfonamide, 4-((2'-amino-4'-methyl-[4,5'-bithiazol]-2-yl)amino)benzenesulfonamide, 4-((2'-amino-4'-methyl-[4,5'-bithiazol]-2-yl)amino)-2-hydroxybenzoic acid, N-(2-((3-hydroxyphenyl)amino)-4'-methyl-[4,5'-bithiazol]-2'-yl)acetamide, 4-((2'-acetamido-4'-methyl-[4,5'-bithiazol]-2-yl)amino)benzoic acid, N-(4-((2'-acetamido-4'-methyl-[4,5'-bithiazol]-2-yl)amino)phenyl)acetamide, N-(2-((4-methoxyphenyl)amino)-4'-methyl-[4,5'-bithiazol]-2'-yl)acetamide, 3-((2'-acetamido-4'-methyl-[4,5'-bithiazol]-2-yl)amino)benzoic acid, N-(2-((4-hydroxyphenyl)amino)-4'-methyl-[4,5'-bithiazol]-2'-yl)acetamide, 4-((4'-methyl-2'-propionamido-[4,5'-bithiazol]-2-yl)amino)benzoic acid, N-(4'-methyl-2-(phenylamino)-[4,5'-bithiazol]-2'-yl)acetamide, and 4-((2'-benzamido-4'-methyl-[4,5'-bithiazol]-2-yl)amino)benzoic acid.

11. Compound for use according to any one of claims 1 to 10, wherein said compound is selected from the group consisting of: 4'-methyl-N2-(4-(trifluoromethyl)phenyl)-[4,5'-bithiazole]-2,2'-diamine, N-(2-((3-hydroxyphenyl)amino)-4'-methyl-[4,5'-bithiazol]-2'-yl)acetamide, N-(4-((2'-acetamido-4'-methyl-[4,5'-bithiazol]-2-yl)amino)phenyl)acetamide, N-(2-((4-methoxyphenyl)amino)-4'-methyl-[4,5'-bithiazol]-2'-yl)acetamide, and N-(4'-methyl-2-(phenylamino)-[4,5'-bithiazol]-2'-yl)acetamide.

12. Compound for use according to any one of claims 1 to 11, wherein the anticancer agent selected from the group consisting of type II topoisomerase inhibitors including topoisomerase II poisons and catalytic inhibitors of topoisomerase II, and type I topoisomerase inhibitors.

13. A pharmaceutical composition which comprises a compound as defined in any one of claims 1 to 11 or a pharmaceutically acceptable salt thereof, another anticancer agent, and a pharmaceutically acceptable carrier, adjuvant or vehicle;
wherein the anticancer agent is selected from the group consisting of type II topoisomerase inhibitors including topoisomerase II poisons and catalytic inhibitors of topoisomerase II, type I topoisomerase inhibitors, Hsp90 inhibitors, proteasome inhibitors, inhibitors of histone deacetylases, protein kinase inhibitors, telomerase inhibitors and microtubule-interfering agents.

14. Combination comprising i) a compound as defined in any one of claims 1 to 11 and ii) another anticancer agent;
wherein the anticancer agent is selected from the group consisting of type II topoisomerase inhibitors including topoisomerase II poisons and catalytic inhibitors of topoisomerase II, type I topoisomerase inhibitors, Hsp90 inhibitors, proteasome inhibitors, inhibitors of histone deacetylases, protein kinase inhibitors, telomerase inhibitors and microtubule-interfering agents.

15. Pharmaceutical composition according to claim 13 or combination according to claim 14, wherein the anticancer agent is selected from the group consisting of type II topoisomerase inhibitors including topoisomerase II poisons and catalytic inhibitors of topoisomerase II, and type I topoisomerase inhibitors.

## Patentansprüche

1. Verbindung der allgemeinen Formel (I) oder ein pharmazeutisch unbedenkliches Salz, Solvat, Amid, Ester, Ether oder N-Oxid davon, 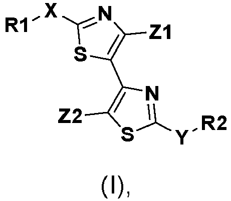
zur Verwendung in Kombination mit einem weiteren Antikrebsmittel bei der Behandlung von Krebs;
wobei:
R1 Wasserstoff, -C(O)R1', substituiertes oder unsubstituiertes C₁₋₆-Alkyl, substituiertes oder unsubstituiertes C₂₋₆-Alkenyl, substituiertes oder unsubstituiertes C₂₋₆-Alkynyl, substituiertes oder unsubstituiertes Cycloalkyl, substituiertes oder unsubstituiertes Cycloalkenyl, substituiertes oder unsubstituiertes Aryl oder substituiertes oder unsubstituiertes Heteroaryl ist;
R1' -OH, substituiertes oder unsubstituiertes C₁₋₆-Alkyl, substituiertes oder unsubstituiertes C₂₋₆-Alkenyl, substituiertes oder unsubstituiertes C₂₋₆-Alkynyl, substituiertes oder unsubstituiertes Cycloalkyl, substituiertes oder unsubstituiertes Cycloalkenyl, substituiertes oder unsubstituiertes Aryl oder substituiertes oder unsubstituiertes Heteroaryl ist;
R2 substituiertes oder unsubstituiertes Aryl oder substituiertes oder unsubstituiertes Heteroaryl ist;
X -O-, -S-, -CH₂- oder -NH- ist;
Y -O-, -S-, -CH₂- oder -NH- ist;
Z1 Wasserstoff, substituiertes oder unsubstituiertes C₁₋₆-Alkyl, substituiertes oder unsubstituiertes C₂₋₆-Alkenyl, substituiertes oder unsubstituiertes C₂₋₆-Alkynyl, substituiertes oder unsubstituiertes Cycloalkyl, substituiertes oder unsubstituiertes Cycloalkenyl, substituiertes oder unsubstituiertes Aryl oder substituiertes oder unsubstituiertes Heteroaryl ist; und
Z2 Wasserstoff, substituiertes oder unsubstituiertes C₁₋₆-Alkyl, substituiertes oder unsubstituiertes C₂₋₆-Alkenyl, substituiertes oder unsubstituiertes C₂₋₆-Alkynyl, substituiertes oder unsubstituiertes Cycloalkyl, substituiertes oder unsubstituiertes Cycloalkenyl, substituiertes oder unsubstituiertes Aryl oder substituiertes oder unsubstituiertes Heteroaryl ist;
wobei das Antikrebsmittel aus der Gruppe ausgewählt ist, die aus Typ-II-Topoisomerase-Inhibitoren, einschließlich Topoisomerase-II-Giften und katalytischer Inhibitoren von Topoisomerase II, Typ-I-Topoisomerase-Inhibitoren, Hsp90-Inhibitoren, Proteasominhibitoren, Inhibitoren von Histondeacetylasen, Proteinkinaseinhibitoren, Telomeraseinhibitoren und mikrotubulistörenden Mitteln besteht.

2. Verbindung zur Verwendung nach Anspruch 1, wobei R2 ein substituiertes oder unsubstituiertes monocyclisches Aryl oder substituiertes oder unsubstituiertes monocyclisches Heteroaryl ist, R2 vorzugsweise ein substituiertes oder unsubstituiertes monocyclisches Aryl ist, R2 stärker bevorzugt ein substituiertes oder unsubstituiertes Phenyl ist.

3. Verbindung zur Verwendung nach Anspruch 1, wobei die Verbindung der Formel (I) eine Verbindung der Formel (II) ist, 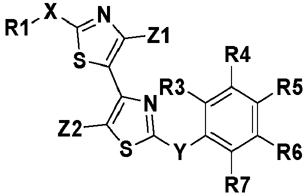 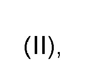 wobei:
R1 Wasserstoff, -C(O)R1', substituiertes oder unsubstituiertes C₁₋₆-Alkyl, substituiertes oder unsubstituiertes C₂₋₆-Alkenyl, substituiertes oder unsubstituiertes C₂₋₆-Alkynyl, substituiertes oder unsubstituiertes Cycloalkyl, substituiertes oder unsubstituiertes Cycloalkenyl, substituiertes oder unsubstituiertes substituiertes oder unsubstituiertes Aryl oder substituiertes oder unsubstituiertes Heteroaryl ist;
R1' -OH, substituiertes oder unsubstituiertes C₁₋₆-Alkyl, substituiertes oder unsubstituiertes C₂₋₆-Alkenyl, substituiertes oder unsubstituiertes C₂₋₆-Alkynyl, substituiertes oder unsubstituiertes Cycloalkyl, substituiertes oder unsubstituiertes Cycloalkenyl, substituiertes oder unsubstituiertes substituiertes oder unsubstituiertes Aryl oder substituiertes oder unsubstituiertes Heteroaryl ist;
R3, R4, R5, R6 und R7 unabhängig aus der Gruppe ausgewählt sind, die aus Wasserstoff, Hydroxyl, substituiertem oder unsubstituiertem C₁₋₆-Alkyl, substituiertem oder unsubstituiertem C₂₋₆-Alkenyl, substituiertem oder unsubstituiertem C₂₋₆-Alkynyl, substituiertem oder unsubstituiertem monocyclischem Cycloalkyl, substituiertem oder unsubstituiertem monocyclischem Cycloalkenyl, substituiertem oder unsubstituiertem monocyclischem Aryl oder substituiertem oder unsubstituiertem monocyclischem Heteroaryl, substituiertem oder unsubstituiertem C₁₋₆-Alkoxy, -C(O)R8, -S(O)2NR9R9' und -NHCOR10 besteht;
R8 -OH oder substituiertes oder unsubstituiertes C₁₋₆-Alkyl, substituiertes oder unsubstituiertes C₂₋₆-Alkenyl, substituiertes oder unsubstituiertes C₂₋₆-Alkynyl, substituiertes oder unsubstituiertes monocyclisches Cycloalkyl, substituiertes oder unsubstituiertes monocyclisches Cycloalkenyl, substituiertes oder unsubstituiertes monocyclisches Aryl oder substituiertes oder unsubstituiertes monocyclisches 5- oder 6-gliedriges Heteroaryl ist, vorzugsweise -OH oder substituiertes oder unsubstituiertes C₁₋₆-Alkyl ist;
R9 Wasserstoff, substituiertes oder unsubstituiertes C₁₋₆-Alkyl, substituiertes oder unsubstituiertes C₂₋₆-Alkenyl, substituiertes oder unsubstituiertes C₂₋₆-Alkynyl, substituiertes oder unsubstituiertes monocyclisches Cycloalkyl, substituiertes oder unsubstituiertes monocyclisches Cycloalkenyl, substituiertes oder unsubstituiertes monocyclisches Aryl oder substituiertes oder unsubstituiertes monocyclisches 5- oder 6-gliedriges Heteroaryl ist, vorzugsweise substituiertes oder unsubstituiertes Oxazolyl ist;
R9' Wasserstoff, substituiertes oder unsubstituiertes C₁₋₆-Alkyl, substituiertes oder unsubstituiertes C₂₋₆-Alkenyl, substituiertes oder unsubstituiertes C₂₋₆-Alkynyl, substituiertes oder unsubstituiertes monocyclisches Cycloalkyl, substituiertes oder unsubstituiertes monocyclisches Cycloalkenyl, substituiertes oder unsubstituiertes monocyclisches Aryl oder substituiertes oder unsubstituiertes monocyclisches 5- oder 6-gliedriges Heteroaryl ist, vorzugsweise Wasserstoff ist;
R10 Wasserstoff, substituiertes oder unsubstituiertes C₁₋₆-Alkyl, substituiertes oder unsubstituiertes C₂₋₆-Alkenyl, substituiertes oder unsubstituiertes C₂₋₆-Alkynyl, substituiertes oder unsubstituiertes monocyclisches Cycloalkyl, substituiertes oder unsubstituiertes monocyclisches Cycloalkenyl, substituiertes oder unsubstituiertes monocyclisches Aryl oder substituiertes oder unsubstituiertes monocyclisches 5- oder 6-gliedriges Heteroaryl ist, vorzugsweise -CH₃ ist;
X -O-, -S-, -CH₂- oder -NH- ist, X vorzugsweise -NH- ist;
Y -O-, -S-, -CH₂- oder -NH- ist, Y vorzugsweise -NH- ist; und
Z1 Wasserstoff, substituiertes oder unsubstituiertes C₁₋₆-Alkyl, substituiertes oder unsubstituiertes C₂₋₆-Alkenyl, substituiertes oder unsubstituiertes C₂₋₆-Alkynyl, substituiertes oder unsubstituiertes Cycloalkyl, substituiertes oder unsubstituiertes Cycloalkenyl, substituiertes oder unsubstituiertes Aryl oder substituiertes oder unsubstituiertes Heteroaryl ist; und
Z2 Wasserstoff, substituiertes oder unsubstituiertes C₁₋₆-Alkyl, substituiertes oder unsubstituiertes C₂₋₆-Alkenyl, substituiertes oder unsubstituiertes C₂₋₆-Alkynyl, substituiertes oder unsubstituiertes Cycloalkyl, substituiertes oder unsubstituiertes Cycloalkenyl, substituiertes oder unsubstituiertes Aryl oder substituiertes oder unsubstituiertes Heteroaryl ist.

4. Verbindung zur Verwendung nach einem der Ansprüche 1 bis 3, wobei Z1 und Z2 jeweils unabhängig Wasserstoff oder substituiertes oder unsubstituiertes C₁₋₆-Alkyl sind.

5. Verbindung zur Verwendung nach Anspruch 1, wobei die Verbindung der Formel (I) eine Verbindung der Formel (III) ist, 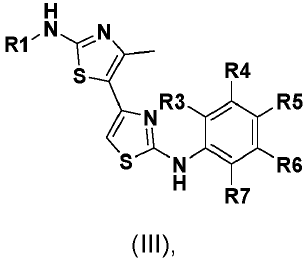 wobei:
R1 Wasserstoff, -C(O)R1', substituiertes oder unsubstituiertes C₁₋₆-Alkyl, substituiertes oder unsubstituiertes C₂₋₆-Alkenyl, substituiertes oder unsubstituiertes C₂₋₆-Alkynyl, substituiertes oder unsubstituiertes Cycloalkyl, substituiertes oder unsubstituiertes Cycloalkenyl, substituiertes oder unsubstituiertes substituiertes oder unsubstituiertes Aryl oder substituiertes oder unsubstituiertes Heteroaryl ist;
R1' -OH, substituiertes oder unsubstituiertes C₁₋₆-Alkyl, substituiertes oder unsubstituiertes C₂₋₆-Alkenyl, substituiertes oder unsubstituiertes C₂₋₆-Alkynyl, substituiertes oder unsubstituiertes Cycloalkyl, substituiertes oder unsubstituiertes Cycloalkenyl, substituiertes oder unsubstituiertes substituiertes oder unsubstituiertes Aryl oder substituiertes oder unsubstituiertes Heteroaryl ist;
R3, R4, R5, R6 und R7 unabhängig aus der Gruppe ausgewählt sind, die aus Wasserstoff, Hydroxyl, substituiertem oder unsubstituiertem C₁₋₆-Alkyl, substituiertem oder unsubstituiertem C₂₋₆-Alkenyl, substituiertem oder unsubstituiertem C₂₋₆-Alkynyl, substituiertem oder unsubstituiertem monocyclischem Cycloalkyl, substituiertem oder unsubstituiertem monocyclischem Cycloalkenyl, substituiertem oder unsubstituiertem monocyclischem Aryl oder substituiertem oder unsubstituiertem monocyclischem Heteroaryl, substituiertem oder unsubstituiertem C₁₋₆-Alkoxy, -C(O)R8, -S(O)₂NR9R9' und -NHCOR10 besteht;
R8 -OH oder substituiertes oder unsubstituiertes C₁₋₆-Alkyl, substituiertes oder unsubstituiertes C₂₋₆-Alkenyl, substituiertes oder unsubstituiertes C₂₋₆-Alkynyl, substituiertes oder unsubstituiertes monocyclisches Cycloalkyl, substituiertes oder unsubstituiertes monocyclisches Cycloalkenyl, substituiertes oder unsubstituiertes monocyclisches Aryl oder substituiertes oder unsubstituiertes monocyclisches 5- oder 6-gliedriges Heteroaryl ist, vorzugsweise -OH oder substituiertes oder unsubstituiertes C₁₋₆-Alkyl ist;
R9 Wasserstoff, substituiertes oder unsubstituiertes C₁₋₆-Alkyl, substituiertes oder unsubstituiertes C₂₋₆-Alkenyl, substituiertes oder unsubstituiertes C₂₋₆-Alkynyl, substituiertes oder unsubstituiertes monocyclisches Cycloalkyl, substituiertes oder unsubstituiertes monocyclisches Cycloalkenyl, substituiertes oder unsubstituiertes monocyclisches Aryl oder substituiertes oder unsubstituiertes monocyclisches 5- oder 6-gliedriges Heteroaryl ist, vorzugsweise substituiertes oder unsubstituiertes Oxazolyl ist;
R9' Wasserstoff, substituiertes oder unsubstituiertes C₁₋₆-Alkyl, substituiertes oder unsubstituiertes C₂₋₆-Alkenyl, substituiertes oder unsubstituiertes C₂₋₆-Alkynyl, substituiertes oder unsubstituiertes monocyclisches Cycloalkyl, substituiertes oder unsubstituiertes monocyclisches Cycloalkenyl, substituiertes oder unsubstituiertes monocyclisches Aryl oder substituiertes oder unsubstituiertes monocyclisches 5- oder 6-gliedriges Heteroaryl ist, vorzugsweise Wasserstoff ist; und
R10 Wasserstoff, substituiertes oder unsubstituiertes C₁₋₆-Alkyl, substituiertes oder unsubstituiertes C₂₋₆-Alkenyl, substituiertes oder unsubstituiertes C₂₋₆-Alkynyl, substituiertes oder unsubstituiertes monocyclisches Cycloalkyl, substituiertes oder unsubstituiertes monocyclisches Cycloalkenyl, substituiertes oder unsubstituiertes monocyclisches Aryl oder substituiertes oder unsubstituiertes monocyclisches 5- oder 6-gliedriges Heteroaryl ist, vorzugsweise -CH₃ ist.

6. Verbindung zur Verwendung nach einem der Ansprüche 3 bis 5, wobei R5 aus der Gruppe ausgewählt ist, die aus Wasserstoff, Hydroxyl, substituiertem oder unsubstituiertem C₁₋₆-Alkyl, substituiertem oder unsubstituiertem C₂₋₆-Alkenyl, substituiertem oder unsubstituiertem C₂₋₆-Alkynyl, substituiertem oder unsubstituiertem monocyclischem Cycloalkyl, substituiertem oder unsubstituiertem monocyclischem Cycloalkenyl, substituiertem oder unsubstituiertem monocyclischem Aryl oder substituiertem oder unsubstituiertem monocyclischem Heteroaryl, substituiertem oder unsubstituiertem C₁₋₆-Alkoxy, -C(O)R8, -S(O)₂NR9R9' und -NHCOR10 besteht;
R8 -OH oder substituiertes oder unsubstituiertes C₁₋₆-Alkyl, substituiertes oder unsubstituiertes C₂₋₆-Alkenyl, substituiertes oder unsubstituiertes C₂₋₆-Alkynyl, substituiertes oder unsubstituiertes monocyclisches Cycloalkyl, substituiertes oder unsubstituiertes monocyclisches Cycloalkenyl, substituiertes oder unsubstituiertes monocyclisches Aryl oder substituiertes oder unsubstituiertes monocyclisches 5- oder 6-gliedriges Heteroaryl ist, vorzugsweise -OH oder substituiertes oder unsubstituiertes C₁₋₆-Alkyl ist;
R9 Wasserstoff, substituiertes oder unsubstituiertes C₁₋₆-Alkyl, substituiertes oder unsubstituiertes C₂₋₆-Alkenyl, substituiertes oder unsubstituiertes C₂₋₆-Alkynyl, substituiertes oder unsubstituiertes monocyclisches Cycloalkyl, substituiertes oder unsubstituiertes monocyclisches Cycloalkenyl, substituiertes oder unsubstituiertes monocyclisches Aryl oder substituiertes oder unsubstituiertes monocyclisches 5- oder 6-gliedriges Heteroaryl ist, vorzugsweise substituiertes oder unsubstituiertes Oxazolyl ist; und
R9' Wasserstoff, substituiertes oder unsubstituiertes C₁₋₆-Alkyl, substituiertes oder unsubstituiertes C₂₋₆-Alkenyl, substituiertes oder unsubstituiertes C₂₋₆-Alkynyl, substituiertes oder unsubstituiertes monocyclisches Cycloalkyl, substituiertes oder unsubstituiertes monocyclisches Cycloalkenyl, substituiertes oder unsubstituiertes monocyclisches Aryl oder substituiertes oder unsubstituiertes monocyclisches 5- oder 6-gliedriges Heteroaryl ist, vorzugsweise Wasserstoff ist.

7. Verbindung zur Verwendung nach einem der Ansprüche 3 bis 6, wobei R5 aus der Gruppe ausgewählt ist, die aus -H, -OH, -CH₃, -CF₃, -COOH, -COCH₃, -SO₂NH₂, -OCH₃, -NHCOCH₃ und 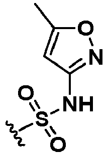 besteht; und/oder
wobei R3, R4, R6 und R7 unabhängig aus der Gruppe ausgewählt sind, die aus -H, -OH, -CH₃, -CF₃, -COOH, -COCH₃, -SO₂NH₂, -OCH₃ und -NHCOCH₃ besteht.

8. Verbindung zur Verwendung nach einem der Ansprüche 3 bis 7, wobei R3, R4, R6 und R7 unabhängig aus der Gruppe ausgewählt sind, die aus -H, -OH, -CH₃, -CF₃, -COOH, -COCH₃, -SO₂NH₂, -OCH₃ und -NHCOCH₃ besteht.

9. Verbindung zur Verwendung nach einem der Ansprüche 3 bis 8, wobei R3 und R4 jeweils -H sind; wobei R6 -H, -OH oder -COOH ist; und/oder wobei R7 -H oder -CH₃ ist.

10. Verbindung zur Verwendung nach einem der Ansprüche 1 bis 9, wobei die Verbindung aus der Gruppe ausgewählt ist, die besteht aus: 4'-Methyl-N2-(4-(trifluormethyl)phenyl)-[4,5'-bithiazol]-2,2'-diamin, 4-((2'-Amino-4'-methyl-[4,5'-bithiazol]-2-yl)amino)-3-methylphenol, 1-(4-((2'-Amino-4'-methyl-[4,5'-bithiazol]-2-yl)amino)phenyl)ethan-1-on, 4-((2'-Amino-4'-methyl-[4,5'-bithiazol]-2-yl)amino)-N-(5-methylisoxazol-3-yl)benzolsulfonamid, 4-((2'-Amino-4'-methyl-[4,5'-bithiazol]-2-yl)amino)benzolsulfonamid, 4-((2'-Amino-4'-methyl-[4,5'-bithiazol]-2-yl)amino)-2-hydroxybenzoesäure, N-(2-((3-Hydroxyphenyl)amino)-4'-methyl-[4,5'-bithiazol]-2'-yl)acetamid, 4-((2'-Acetamido-4'-methyl-[4,5'-bithiazol]-2-yl)amino)benzoesäure, N-(4-((2'-Acetamido-4'-methyl-[4,5'-bithiazol]-2-yl)amino)phenyl)acetamid, N-(2-((4-Methoxyphenyl)amino)-4'-methyl-[4,5'-bithiazol]-2'-yl)acetamid, 3-((2'-Acetamido-4'-methyl-[4,5'-bithiazol]-2-yl)amino)benzoesäure, N-(2-((4-Hydroxyphenyl)amino)-4'-methyl-[4,5'-bithiazol]-2'-yl)acetamid, 4-((4'-Methyl-2'-propionamido-[4,5'-bithiazol]-2-yl)amino)benzoesäure, N-(4'-Methyl-2-(phenylamino)-[4,5'-bithiazol]-2'-yl)acetamid und 4-((2'-Benzamido-4'-methyl-[4,5'-bithiazol]-2-yl)amino)benzoesäure.

11. Verbindung zur Verwendung nach einem der Ansprüche 1 bis 10, wobei die Verbindung aus der Gruppe ausgewählt ist, die besteht aus: 4'-Methyl-N2-(4-(trifluormethyl)phenyl)-[4,5'-bithiazol]-2,2'-diamin, N-(2-((3-Hydroxyphenyl)amino)-4'-methyl-[4,5'-bithiazol]-2'-yl)acetamid, N-(4-((2'-Acetamido-4'-methyl-[4,5'-bithiazol]-2-yl)amino)phenyl)acetamid, N-(2-((4-Methoxyphenyl)amino)-4'-methyl-[4,5'-bithiazol]-2'-yl)acetamid und N-(4'-Methyl-2-(phenylamino)-[4,5'-bithiazol]-2'-yl)acetamid.

12. Verbindung zur Verwendung nach einem der Ansprüche 1 bis 11, wobei das Antikrebsmittel aus der Gruppe ausgewählt ist, die aus Typ-II-Topoisomerase-Inhibitoren, einschließlich Topoisomerase-II-Giften und katalytischer Inhibitoren von Topoisomerase II, und Typ-I-Topoisomerase-Inhibitoren besteht.

13. Pharmazeutische Zusammensetzung, die eine Verbindung nach einem der Ansprüche 1 bis 11 oder ein pharmazeutisch unbedenkliches Salz davon, ein weiteres Antikrebsmittel und einen pharmazeutisch unbedenklichen Träger, ein pharmazeutisch unbedenkliches Adjuvans oder ein pharmazeutisch unbedenkliches Vehikel umfasst;
wobei das Antikrebsmittel aus der Gruppe ausgewählt ist, die aus Typ-II-Topoisomerase-Inhibitoren, einschließlich Topoisomerase-II-Giften und katalytischer Inhibitoren von Topoisomerase II, Typ-I-Topoisomerase-Inhibitoren, Hsp90-Inhibitoren, Proteasominhibitoren, Inhibitoren von Histondeacetylasen, Proteinkinaseinhibitoren, Telomeraseinhibitoren und mikrotubulistörenden Mitteln besteht.

14. Kombination, die i) eine Verbindung nach einem der Ansprüche 1 bis 11 und ii) ein weiteres Antikrebsmittel umfasst.
wobei das Antikrebsmittel aus der Gruppe ausgewählt ist, die aus Typ-II-Topoisomerase-Inhibitoren, einschließlich Topoisomerase-II-Giften und katalytischer Inhibitoren von Topoisomerase II, Typ-I-Topoisomerase-Inhibitoren, Hsp90-Inhibitoren, Proteasominhibitoren, Inhibitoren von Histondeacetylasen, Proteinkinaseinhibitoren, Telomeraseinhibitoren und mikrotubulistörenden Mitteln besteht.

15. Pharmazeutische Zusammensetzung nach Anspruch 13 oder Kombination nach Anspruch 14, wobei das Antikrebsmittel aus der Gruppe ausgewählt ist, die aus Typ-II-Topoisomerase-Inhibitoren, einschließlich Topoisomerase-II-Giften und katalytischer Inhibitoren von Topoisomerase II, und Typ-I-Topoisomerase-Inhibitoren besteht.

## Revendications

1. Composé de formule générale (I), ou un sel, solvate, amide, ester, éther ou N-oxyde pharmaceutiquement acceptable de celui-ci, 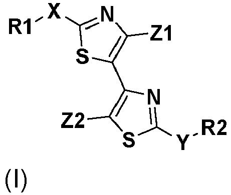
pour une utilisation en combinaison avec un autre agent anticancéreux dans le traitement d'un cancer ;
dans lequel
R1 est hydrogène, -C(O)R1', alkyle en C₁₋₆ substitué ou non substitué, alcényle en C₂₋₆ substitué ou non substitué, alcynyle en C₂₋₆substitué ou non substitué, cycloalkyle substitué ou non substitué, cycloalcényle substitué ou non substitué, aryle substitué ou non substitué ou hétéroaryle substitué ou non substitué ;
R1 est -OH, alkyle en C₁₋₆ substitué ou non substitué, alcényle en C₂₋₆ substitué ou non substitué, alcynyle en C₂₋₆substitué ou non substitué, cycloalkyle substitué ou non substitué, cycloalcényle substitué ou non substitué, aryle substitué ou non substitué ou hétéroaryle substitué ou non substitué ;
R2 est aryle substitué ou non substitué ou hétéroaryle substitué ou non substitué ;
X est -O-, -S-, -CH₂- ou -NH- ;
Y est -O-, -S-, -CH₂- ou -NH- ;
Z1 est hydrogène, alkyle en C₁₋₆ substitué ou non substitué, alcényle en C₂₋₆ substitué ou non substitué, alcynyle en C₂₋₆substitué ou non substitué, cycloalkyle substitué ou non substitué, cycloalcényle substitué ou non substitué, aryle substitué ou non substitué ou hétéroaryle substitué ou non substitué ; et
Z2 est hydrogène, alkyle en C₁₋₆ substitué ou non substitué, alcényle en C₂₋₆ substitué ou non substitué, alcynyle en C₂₋₆substitué ou non substitué, cycloalkyle substitué ou non substitué, cycloalcényle substitué ou non substitué, aryle substitué ou non substitué ou hétéroaryle substitué ou non substitué ;
dans lequel l'agent anticancéreux est choisi dans le groupe constitué des inhibiteurs de topoisomérase de type II, y compris les poisons de topoisomérase II et les inhibiteurs catalytiques de topoisomérase II, des inhibiteurs de topoisomérase de type I, des inhibiteurs de Hsp90, des inhibiteurs de protéasome, des inhibiteurs d'histone désacétylases, des inhibiteurs de protéine kinase, des inhibiteurs de télomérase et des agents d'interférence avec les microtubules.

2. Composé destiné à être utilisé selon la revendication 1, dans lequel R2 est un aryle monocyclique substitué ou non substitué ou un hétéroaryle monocyclique substitué ou non substitué, de préférence R2 est un aryle monocyclique substitué ou non substitué, plus préférablement R2 est un phényle substitué ou non substitué.

3. Composé destiné à être utilisé selon la revendication 1, dans lequel le composé de formule (I) est un composé de formule (II) 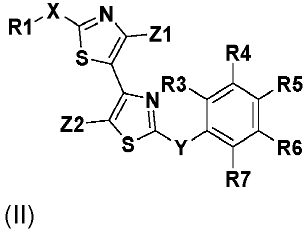 dans lequel
R1 est hydrogène, -C(O)R1', alkyle en C₁₋₆ substitué ou non substitué, alcényle en C₂₋₆ substitué ou non substitué, alcynyle en C₂₋₆substitué ou non substitué, cycloalkyle substitué ou non substitué, cycloalcényle substitué ou non substitué, aryle substitué ou non substitué ou hétéroaryle substitué ou non substitué ;
R1' est -OH, alkyle en C₁₋₆ substitué ou non substitué, alcényle en C₂₋₆ substitué ou non substitué, alcynyle en C₂₋₆substitué ou non substitué, cycloalkyle substitué ou non substitué, cycloalcényle substitué ou non substitué, aryle substitué ou non substitué ou hétéroaryle substitué ou non substitué ;
R3, R4, R5, R6 et R7 sont indépendamment sélectionnés dans le groupe consistant en hydrogène, hydroxyle, alkyle en C₁₋₆ substitué ou non substitué, alcényle en C₂₋₆ substitué ou non substitué, alcynyle en C₂₋₆substitué ou non substitué, cycloalkyle monocyclique substitué ou non substitué, cycloalcényle monocyclique substitué ou non substitué, aryle monocyclique substitué ou non substitué ou hétéroaryle monocyclique substitué ou non substitué, alcoxy en C₁₋₆ substitué ou non substitué, -C(O)R8, - S(O)2NR9R9' et -NHCOR10 ; R8 est -OH ou alkyle en C₁₋₆ substitué ou non substitué, alcényle en C₂₋₆ substitué ou non substitué, alcynyle en C₂₋₆substitué ou non substitué, cycloalkyle monocyclique substitué ou non substitué, cycloalcényle monocyclique substitué ou non substitué, aryle monocyclique substitué ou non substitué ou hétéroaryle monocyclique substitué ou non substitué à 5 ou 6 chaînons, est de préférence -OH ou alkyle en C₁₋₆ substitué ou non substitué ;
R9 est hydrogène, alkyle en C₁₋₆ substitué ou non substitué, alcényle en C₂₋₆ substitué ou non substitué, alcynyle en C₂₋₆substitué ou non substitué, cycloalkyle monocyclique substitué ou non substitué, cycloalcényle monocyclique substitué ou non substitué, aryle monocyclique substitué ou non substitué ou hétéroaryle monocyclique substitué ou non substitué à 5 ou 6 chaînons, est de préférence oxazolyle substitué ou non substitué ;
R9' est hydrogène, alkyle en C₁₋₆ substitué ou non substitué, alcényle en C₂₋₆ substitué ou non substitué, alcynyle en C₂₋₆substitué ou non substitué, cycloalkyle monocyclique substitué ou non substitué, cycloalcényle monocyclique substitué ou non substitué, aryle monocyclique substitué ou non substitué ou hétéroaryle monocyclique substitué ou non substitué à 5 ou 6 chaînons, est de préférence hydrogène ;
R10 est hydrogène, alkyle en C₁₋₆ substitué ou non substitué, alcényle en C₂₋₆ substitué ou non substitué, alcynyle en C₂₋₆substitué ou non substitué, cycloalkyle monocyclique substitué ou non substitué, cycloalcényle monocyclique substitué ou non substitué, aryle monocyclique substitué ou non substitué ou hétéroaryle monocyclique substitué ou non substitué à 5 ou 6 chaînons, est de préférence -CH_{3;}
X est -O-, -S-, -CH₂- ou -NH-, de préférence X est -NH- ;
Y est -O-, -S-, -CH₂- ou -NH-, de préférence Y est -NH- ; et
Z1 est hydrogène, alkyle en C₁₋₆ substitué ou non substitué, alcényle en C₂₋₆ substitué ou non substitué, alcynyle en C₂₋₆substitué ou non substitué, cycloalkyle substitué ou non substitué, cycloalcényle substitué ou non substitué, aryle substitué ou non substitué ou hétéroaryle substitué ou non substitué ; et
Z2 est hydrogène, alkyle en C₁₋₆ substitué ou non substitué, alcényle en C₂₋₆ substitué ou non substitué, alcynyle en C₂₋₆substitué ou non substitué, cycloalkyle substitué ou non substitué, cycloalcényle substitué ou non substitué, aryle substitué ou non substitué ou hétéroaryle substitué ou non substitué.

4. Composé destiné à être utilisé selon l'une quelconque des revendications 1 à 3, dans lequel Z1 et Z2 sont chacun indépendamment un hydrogène ou un alkyle en C₁₋₆ substitué ou non substitué.

5. Composé destiné à être utilisé selon la revendication 1, dans lequel le composé de formule (I) est un composé de formule (III) 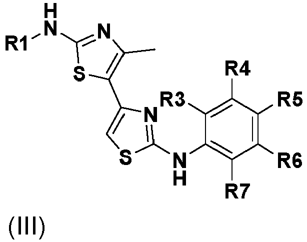 dans lequel
R1 est hydrogène, -C(O)R1', alkyle en C₁₋₆ substitué ou non substitué, alcényle en C₂₋₆ substitué ou non substitué, alcynyle en C₂₋₆substitué ou non substitué, cycloalkyle substitué ou non substitué, cycloalcényle substitué ou non substitué, aryle substitué ou non substitué ou hétéroaryle substitué ou non substitué ;
R1' est -OH, alkyle en C₁₋₆ substitué ou non substitué, alcényle en C₂₋₆ substitué ou non substitué, alcynyle en C₂₋₆substitué ou non substitué, cycloalkyle substitué ou non substitué, cycloalcényle substitué ou non substitué, aryle substitué ou non substitué ou hétéroaryle substitué ou non substitué ;
R3, R4, R5, R6 et R7 sont indépendamment sélectionnés dans le groupe consistant en hydrogène, hydroxyle, alkyle en C₁₋₆ substitué ou non substitué, alcényle en C₂₋₆ substitué ou non substitué, alcynyle en C₂₋₆substitué ou non substitué, cycloalkyle monocyclique substitué ou non substitué, cycloalcényle monocyclique substitué ou non substitué, aryle monocyclique substitué ou non substitué ou hétéroaryle monocyclique substitué ou non substitué, alcoxy en C₁₋₆ substitué ou non substitué, -C(O)R8, -S(O)₂NR9R9' et -NHCOR10 ;
R8 est -OH ou alkyle en C₁₋₆ substitué ou non substitué, alcényle en C₂₋₆ substitué ou non substitué, alcynyle en C₂₋₆substitué ou non substitué, cycloalkyle monocyclique substitué ou non substitué, cycloalcényle monocyclique substitué ou non substitué, aryle monocyclique substitué ou non substitué ou hétéroaryle monocyclique substitué ou non substitué à 5 ou 6 chaînons, est de préférence -OH ou alkyle en C₁₋₆ substitué ou non substitué ;
R9 est hydrogène, alkyle en C₁₋₆ substitué ou non substitué, alcényle en C₂₋₆ substitué ou non substitué, alcynyle en C₂₋₆substitué ou non substitué, cycloalkyle monocyclique substitué ou non substitué, cycloalcényle monocyclique substitué ou non substitué, aryle monocyclique substitué ou non substitué ou hétéroaryle monocyclique substitué ou non substitué à 5 ou 6 chaînons, est de préférence oxazolyle substitué ou non substitué ;
R9' est hydrogène, alkyle en C₁₋₆ substitué ou non substitué, alcényle en C₂₋₆ substitué ou non substitué, alcynyle en C₂₋₆substitué ou non substitué, cycloalkyle monocyclique substitué ou non substitué, cycloalcényle monocyclique substitué ou non substitué, aryle monocyclique substitué ou non substitué ou hétéroaryle monocyclique substitué ou non substitué à 5 ou 6 chaînons, est de préférence hydrogène ; et
R10 est hydrogène, alkyle en C₁₋₆ substitué ou non substitué, alcényle en C₂₋₆ substitué ou non substitué, alcynyle en C₂₋₆substitué ou non substitué, cycloalkyle monocyclique substitué ou non substitué, cycloalcényle monocyclique substitué ou non substitué, aryle monocyclique substitué ou non substitué ou hétéroaryle monocyclique substitué ou non substitué à 5 ou 6 chaînons, est de préférence -CH₃.

6. Composé destiné à être utilisé selon l'une quelconque des revendications 3 à 5, dans lequel R5 est choisi dans le groupe consistant en hydrogène, hydroxyle, alkyle en C₁₋₆ substitué ou non substitué, alcényle en C₂₋₆ substitué ou non substitué, alcynyle en C₂₋₆ substitué ou non substitué, cycloalkyle monocyclique substitué ou non substitué, cycloalcényle monocyclique substitué ou non substitué, aryle monocyclique substitué ou non substitué ou hétéroaryle monocyclique substitué ou non substitué, alcoxy en C₁₋₆ substitué ou non substitué, -C(O)R8, -S(O)₂NR9R9' et -NHCOR10 ;
R8 est -OH ou alkyle en C₁₋₆ substitué ou non substitué, alcényle en C₂₋₆ substitué ou non substitué, alcynyle en C₂₋₆substitué ou non substitué, cycloalkyle monocyclique substitué ou non substitué, cycloalcényle monocyclique substitué ou non substitué, aryle monocyclique substitué ou non substitué ou hétéroaryle monocyclique substitué ou non substitué à 5 ou 6 chaînons, est de préférence -OH ou alkyle en C₁₋₆ substitué ou non substitué ;
R9 est hydrogène, alkyle en C₁₋₆ substitué ou non substitué, alcényle en C₂₋₆ substitué ou non substitué, alcynyle en C₂₋₆substitué ou non substitué, cycloalkyle monocyclique substitué ou non substitué, cycloalcényle monocyclique substitué ou non substitué, aryle monocyclique substitué ou non substitué ou hétéroaryle monocyclique substitué ou non substitué à 5 ou 6 chaînons, est de préférence oxazolyle substitué ou non substitué ; et
R9' est hydrogène, alkyle en C₁₋₆ substitué ou non substitué, alcényle en C₂₋₆ substitué ou non substitué, alcynyle en C₂₋₆substitué ou non substitué, cycloalkyle monocyclique substitué ou non substitué, cycloalcényle monocyclique substitué ou non substitué, aryle monocyclique substitué ou non substitué ou hétéroaryle monocyclique substitué ou non substitué à 5 ou 6 chaînons, est de préférence hydrogène.

7. Composé destiné à être utilisé selon l'une quelconque des revendications 3 à 6, dans lequel R5 est choisi dans le groupe consistant en -H, -OH, -CH₃, - CF₃, -COOH, -COCH₃, -SO₂NH₂, -OCH₃, -NHCOCH₃ et 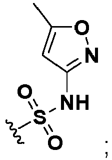 et/ou dans lequel R3, R4, R6 et R7 sont choisis indépendamment dans le groupe consistant en -H, -OH, -CH₃, -CF₃, -COOH, -COCH₃, -SO₂NH₂, -OCH₃ et - NHCOCH₃.

8. Composé destiné à être utilisé selon l'une quelconque des revendications 3 à 7, dans lequel R3, R4, R6 et R7 sont choisis indépendamment dans le groupe consistant en-H, -OH, -CH₃, -CF₃, -COOH, -COCH₃, -SO₂NH₂, -OCH₃ et -NHCOCH₃.

9. Composé destiné à être utilisé selon l'une quelconque des revendications 3 à 8, dans lequel R3 et R4 sont chacun -H; dans lequel R6 est -H, -OH ou - COOH; et / ou dans lequel R7 est -H or -CH₃.

10. Composé destiné à être utilisé selon l'une quelconque des revendications 1 à 9, dans lequel ledit composé est choisi parmi le groupe constitué par: 4'-méthyl-N2-(4-(trifluorométhyl)phényl)-[4,5'-bithiazole]-2,2'-diamine, 4-((2'-amino-4'-méthyl-[4,5'-bithiazol]-2-yl)amino)-3-méthylphenol, 1-(4-((2'-amino-4'-méthyl-[4,5'-bithiazol]-2-yl)amino)phényl)éthan-1-one, 4-((2'-amino-4'-méthyl-[4,5'-bithiazol]-2-yl)amino)-N-(5-méthylisoxazol-3-yl)benzènesulfona-mide, 4-((2'-amino-4'-méthyl-[4,5'-bithiazol]-2-yl)amino)benzènesulfonamide, acide 4-((2'-amino-4'-méthyl-[4,5'-bithiazol]-2-yl)amino)-2-hydroxybenzoïque, N-(2-((3-hydroxyphényl)amino)-4'-méthyl-[4,5'-bithiazol]-2'-yl)acétamide, acide 4-((2'-acétamido-4'-méthyl-[4,5'-bithiazol]-2-yl)amino)benzoïque, N-(4-((2'-acétamido-4'-méthyl-[4,5'-bithiazol]-2-yl)amino)phényl)acétamide, N-(2-((4-méthoxyphényl)amino)-4'-méthyl-[4,5'-bithiazol]-2'-yl)acétamide, acide 3-((2'-acétamido-4'-méthyl-[4,5'-bithiazol]-2-yl)amino)benzoïque, N-(2-((4-hydroxyphényl)amino)-4'-méthyl-[4,5'-bithiazol]-2'-yl)acétamide, acide 4-((4'-méthyl-2'-propionamido-[4,5'-bithiazol]-2-yl)amino)benzoïque, N-(4'-méthyl-2-(phénylamino)-[4,5'-bithiazol]-2'-yl)acétamide et acide 4-((2'-benzamido-4'-méthyl-[4,5'-bithiazol]-2-yl)amino)benzoïque.

11. Composé destiné à être utilisé selon l'une quelconque des revendications 1 à 10, dans lequel ledit composé est choisi parmi le groupe constitué par: 4'-méthyl-N2-(4-(trifluorométhyl)phényl)-[4,5'-bithiazole]-2,2'-diamine, N-(2-((3-hydroxyphényl)amino)-4'-méthyl-[4,5'-bithiazol]-2'-yl)acétamide, N-(4-((2'-acétamido-4'-méthyl-[4,5'-bithiazol]-2-yl)amino)phényl)acétamide, N-(2-((4-méthoxyphényl)amino)-4'-méthyl-[4,5'-bithiazol]-2'-yl)acétamide et N-(4'-méthyl-2-(phénylamino)-[4,5'-bithiazol]-2'-yl)acétamide.

12. Composé destiné à être utilisé selon l'une quelconque des revendications 1 à 11, dans lequel l'agent anticancéreux choisi dans le groupe constitué par les inhibiteurs de topoisomérase de type II, y compris les poisons de topoisomérase II et les inhibiteurs catalytiques de topoisomérase II et les inhibiteurs de topoisomérase de type I.

13. Composition pharmaceutique qui comprend un composé tel que défini dans l'une quelconque des revendications 1 à 11 ou un sel pharmaceutiquement acceptable de celui-ci, un autre agent anticancéreux et un support, adjuvant ou véhicule pharmaceutiquement acceptable ;
dans laquelle l'agent anticancéreux est choisi dans le groupe constitué des inhibiteurs de topoisomérase de type II, y compris les poisons de topoisomérase II et les inhibiteurs catalytiques de topoisomérase II, des inhibiteurs de topoisomérase de type I, des inhibiteurs de Hsp90, des inhibiteurs de protéasome, des inhibiteurs d'histone désacétylases, des inhibiteurs de protéine kinase, des inhibiteurs de télomérase et des agents d'interférence avec les microtubules.

14. Combinaison comprenant i) un composé tel que défini dans l'une quelconque des revendications 1 à 11 et ii) un autre agent anticancéreux ;
dans laquelle l'agent anticancéreux est choisi dans le groupe constitué des inhibiteurs de topoisomérase de type II, y compris les poisons de topoisomérase II et les inhibiteurs catalytiques de topoisomérase II, des inhibiteurs de topoisomérase de type I, des inhibiteurs de Hsp90, des inhibiteurs de protéasome, des inhibiteurs d'histone désacétylases, des inhibiteurs de protéine kinase, des inhibiteurs de télomérase et des agents d'interférence avec les microtubules.

15. Composition pharmaceutique selon la revendication 13 ou combinaison selon la revendication 14, dans laquelle l'agent anticancéreux choisi dans le groupe constitué par les inhibiteurs de topoisomérase de type II, y compris les poisons de topoisomérase II et les inhibiteurs catalytiques de topoisomérase II et les inhibiteurs de topoisomérase de type I.
